# EUROPEAN PATENT APPLICATION

(11) **EP 4 219 548 A1**
(43) Date of publication of application: **02.08.2023**
(21) Application number: 21872574.5
(22) Date of filing: 24.09.2021
(51) Int. Cl.: C07K 16/10, A61K 39/395, A61P 31/14, A61P 43/00, C07K 16/28, C12N 15/13

(54) **SARS-COV-2 NEUTRALIZING ANTIBODY OR FRAGMENT THEREOF**

(30) Priority: 25.09.2020 JP 2020161205; 28.01.2021 JP 2021012394
(71) Applicant: Keio University, Tokyo, 108-8345 (JP); Japan as Represented by Director-General of National Institute of Infectious Diseases, Tokyo 162-8640 (JP); RIKEN, Wako-shi, Saitama 351-0198 (JP); SHIGA UNIVERSITY OF MEDICAL SCIENCE, Otsu-shi Shiga 520-2192 (JP); The University of Tokyo, Bunkyo-ku, Tokyo 113-8654 (JP)
(72) Inventor: TAKESHITA Masaru, Tokyo 160-8582 (JP); TAKEUCHI Tsutomu, Tokyo 160-8582 (JP); SUZUKI Katsuya, Tokyo 160-8582 (JP); SAYA Hideyuki, Tokyo 160-8582 (JP); TAKAHASHI Yoshimasa, Tokyo 162-8640 (JP); MORIYAMA Saya, Tokyo 162-8640 (JP); FUKUYAMA Hidehiro, Wako-shi, Saitama 351-0198 (JP); OKAMURA Chieko, Wako-shi, Saitama 351-0198 (JP); SHIROUZU Mikako, Wako-shi, Saitama 351-0198 (JP); MATSUMOTO Takehisa, Wako-shi, Saitama 351-0198 (JP); KAMADA Katsuhiko, Wako-shi, Saitama 351-0198 (JP); ITOH Yasushi, Otsu-shi, Shiga 520-2192 (JP); ISHIGAKI Hirohito, Otsu-shi, Shiga 520-2192 (JP); NAKAYAMA Misako, Otsu-shi, Shiga 520-2192 (JP); KITAGAWA Yoshinori, Otsu-shi, Shiga 520-2192 (JP); KAWAOKA Yoshihiro, Tokyo 113-8654 (JP)
(74) Representative: Cabinet Becker et Associés
(86) International application number: PCT/JP2021/035159
(87) International publication number: WO 2022/065445

(57) **Abstract**

An antibody against spike protein of SARS-CoV-2 is provided, the antibody having a specific heavy chain variable region and a specific light chain variable region, or a fragment of the antibody, the antibody or fragment thereof inhibiting the binding between the spike protein of SARS-CoV-2 and ACE2, the antibody or fragment thereof inhibiting SARS-CoV-2 infection; and a pharmaceutical composition including the antibody or fragment thereof and a pharmaceutically acceptable carrier, the pharmaceutical composition including two or more kinds of the antibody or fragment thereof.

## Description

### [Technical Field]

The present invention relates to a SARS-CoV-2 neutralizing antibody or a fragment thereof and a pharmaceutical composition. Priority is claimed on Japanese Patent Application No. 2020-161205, filed September 25, 2020, and Japanese Patent Application No. 2021-012394, filed January 28, 2021, the contents of which are incorporated herein by reference.

### [Background Art]

Antibody drugs, which are one type of molecular targeted therapy, were initially developed by targeting molecules that are highly expressed in malignant tumors (CD20 and the like), and targeting cytokines (TNFα and the like) that play a central role in the area of rheumatism and collagen diseases. At first, chimeric antibodies in which the antigen-recognizing moiety of an antibody produced in mice was introduced into the basic skeleton of human IgG (rituximab, infliximab, and the like) were developed. Subsequently, to further reduce antigenicity, humanized antibodies in which all sequences were rearranged into sequences derived from humans were produced, and are actually used for treatment. Since antibody drugs do not respond to molecules other than the target molecules, the most important feature is that they have few side effects other than allergies, which inevitably occur on rare occasions. Development of antibody drugs is in progress for many molecules, causing a paradigm shift in the treatment of malignant tumors and inflammatory diseases. These antibody drugs target molecules in the human living body, and essentially, antibodies are not produced against self-components. For this reason, antibodies cannot be obtained directly from humans, and a technique of immunizing an experimental animal with a target molecule, obtaining an antibody and then humanizing the antibody is mainly used.

On the other hand, in normal infectious diseases, since exogenous antigens that do not exist in the human living body invade, antibodies are produced as an immune response to those antigens, which are believed to work in the clearance of pathogens and prevention of subsequent infections. Utilizing this mechanism, in recent years, a development of extracting an antibody component carried by affected patients against an intractable and highly pathogenic viral infection such as Ebola virus and using the antibody component as an antibody drug has been carried out (see Non-Patent Document 1). When the development of such an antibody drug is carried out, it is expected that the antibody drug will be effective as a therapeutic drug by preventing the virus from infecting cells with an antibody that targets a receptor-binding domain on the surface of the virus when infecting human cells.

In infection immunity, since immunological memory remains in patients who have recovered, memory B cells in the peripheral blood of the patients include cells that remember antibodies against the viruses. Among them, cells that produce an antiviral antibody are selected using a recombinant virus-derived protein (including a receptor-binding domain) as "bait", the variable region of the antibody of each cell is acquired using single-cell technology, and a product produced in vitro from the variable region as a monoclonal antibody is developed as a therapeutic drug candidate.

With regard to COVID-19, which is an infectious disease caused by SARS-CoV-2 virus that has spread since the end of 2019, it has been revealed that the spike protein on the virus surface binds to the ACE2 receptor on the surface of human cells to establish infection. The spike protein is a single-pass transmembrane protein, and it has been identified that the extracellular region is divided into two domains, namely, S1 on the free end side and S2 on the cell membrane side, and the protein has a receptor-binding domain (RBD) in S1 (Non-Patent Document 2).

Non-Patent Document 3 discloses a technology for producing a monoclonal antibody from human-derived peripheral blood B cells using an antigen as "bait". Non-Patent Document 4 by the present inventors discloses a technique for efficiently producing IgG from human antibody-producing cells. Non-Patent Document 5 discloses a method for constructing cDNA libraries from single cells.

Non-Patent Documents 6 to 12 disclose the production of monoclonal antibodies from COVID-19 patients, and the methods, conditions, and evaluation indices for the neutralization test are different in each case. Non-Patent Document 6 discloses an antibody acquired from patient-derived B cells using RBD as "bait", and the antibody concentration that can be completely neutralized in a neutralization test using live viruses is about 1 to 10 µg/mL. Non-Patent Document 7 discloses an antibody acquired from patient-derived B cells using the spike protein extracellular domain as "bait", and the antibody concentration that can be completely neutralized in a neutralization test using live viruses is about 1 to 10 µg/mL. Non-Patent Document 8 discloses an antibody acquired from patient-derived B cells using both the RBD and the spike protein as "bait", the IC50 in a neutralization test using a pseudovirus is about 0.001 to 0.01 µg/mL, and an in vivo effect was confirmed in animal experiments. Non-Patent Document 9 discloses an antibody acquired from patient-derived B cells using the RBD as "bait", and the antibody concentration that can be completely neutralized in a neutralization test using live viruses is about 1 to 10 µg/mL. Non-Patent Document 10 discloses an antibody acquired from patient-derived B cells using a trimeric spike protein as "bait", and an antibody concentration that can be completely neutralized in a neutralization test using live viruses is about 1 to 10 µg/mL. Non-Patent Document 11 discloses an antibody acquired from patient-derived B cells using a trimeric spike protein as "bait", the antibody concentration that can be completely neutralized in a neutralization test using live viruses is about 0.1 to 1 µg/mL, and the effect was confirmed in vivo as a result of animal experiments. Non-Patent Document 12 discloses an antibody acquired from patient-derived B cells using a trimeric spike protein as "bait", and the antibody concentration that can be completely neutralized in a neutralization test using live viruses is about 0.01 to 0.1 µg/mL.

### [Citation List]

### [Non-Patent Documents]

[Non-Patent Document 1]
   Martin R Gaudinski, et al., Safety, tolerability, pharmacokinetics, and immunogenicity of the therapeutic monoclonal antibody mAb114 targeting Ebola virus glycoprotein (VRC 608): an open-label phase 1 study, Lancet, 393 (10174), 889-898, 2019.
[Non-Patent Document 2]
   Peng Zhou, et al., A pneumonia outbreak associated with a new coronavirus of probable bat origin, Nature, 579, 270-273, 2020.
[Non-Patent Document 3]
   Jenna J. Guthmiller, et al., An Efficient Method to Generate Monoclonal Antibodies from Human B Cells, Methods Mol Biol., 1904, 109-145, 2019.
[Non-Patent Document 4]
   Masaru Takeshita, et al., Antigen-driven selection of antibodies against SSA, SSB and the centromere 'complex', including a novel antigen, MIS12 complex, in human salivary glands, Ann Rheum Dis., 79 (1), 150-158, 2020.
[Non-Patent Document 5]
   John J. Trombetta, et al., Preparation of Single-Cell RNA-Seq Libraries for Next Generation Sequencing, Curr Protoc Mol Biol., 107, 4.22.1-4.22.17, 2014.
[Non-Patent Document 6]
   Rui Shi, et al., A human neutralizing antibody targets the receptor-binding site of SARS-CoV-2, Nature, 584, 120-124, 2020.
[Non-Patent Document 7]
   Xiangyang Chi, et al., A neutralizing human antibody binds to the N-terminal domain of the Spike protein of SARS-CoV-2, Science, 369 (6504), 650-655, 2020.
[Non-Patent Document 8]
   Thomas F Rogers, et al., Isolation of potent SARS-CoV-2 neutralizing antibodies and protection from disease in a small animal model, Science, 369 (6506), 956-963, 2020.
[Non-Patent Document 9]
   Bin Ju, et al., Human neutralizing antibodies elicited by SARS-CoV-2 infection, Nature, 584, 115-119, 2020.
[Non-Patent Document 10]
   Seth J. Zost, et al., Rapid isolation and profiling of a diverse panel of human monoclonal antibodies targeting the SARS-CoV-2 spike protein, Nature Medicine, 26, 1422-1427, 2020.
[Non-Patent Document 11]
   Seth J. Zost, et al., Potently neutralizing and protective human antibodies against SARS-CoV-2, Nature, 584, 443-449, 2020.
[Non-Patent Document 12]
   Lihong Liu, et al., Potent neutralizing antibodies against multiple epitopes on SARS-CoV-2 spike, Nature, 584, 450-456, 2020.

### [Summary of Invention]

### [Technical Problem]

As described above, a large number of antibodies having neutralizing ability for SARS-CoV-2 have been developed. Generally, antibodies with a high affinity for antigens are considered to be superior; however, the ability to neutralize viral infection is associated not only with simple affinity but also with various problems such as whether epitopes can be inhibited in terms of steric structure since epitopes are important sites for binding to receptors, and the neutralizing ability can only be confirmed by actually conducting a neutralization test using a virus. In the above-mentioned documents, a neutralization test is carried out using a distinctive technique in each case. Currently, no standard technique for measuring the neutralizing ability against SARS-CoV-2 has been established, and since the numerical value can fluctuate depending on the experimental conditions, it is difficult to compare the values, and the issue of which antibody is superior has not been identified. Furthermore, even in the case where efficacy and safety have been recognized at the animal level, it is not clear whether efficacy can be confirmed in humans, and the possibility of having side effects due to immunogenicity, cross-reaction, and the like when administered cannot be ruled out. Therefore, in order to develop excellent antibody drugs, there is no choice but to produce a large number of antibodies and select the best ones through trial and error.

By producing a large number of new antibodies having a high neutralizing ability and conducting neutralization tests using techniques that are considered as international standard methods, it is possible to identify neutralizing antibodies that exhibit excellent effects. Thus, it is an object of the invention to develop a new neutralizing antibody, in conjunction with the National Institute of Infectious Diseases, which is conducting international standardization of neutralization tests.

### [Solution to Problem]

The present invention includes the following embodiments.
[1] An antibody against spike protein of SARS-CoV-2, or a fragment of the antibody, the antibody or fragment thereof having a heavy chain variable region and a light chain variable region described in any one of the following items (1) to (26):
   (1) a heavy chain variable region in which complementarity-determining regions (CDR) 1, CDR2, and CDR3 determined according to a Kabat numbering system include amino acid sequences set forth in SEQ ID NO:1, SEQ ID NO:2, and SEQ ID NO:3, respectively, and a light chain variable region in which CDR1, CDR2, and CDR3 determined according to the Kabat numbering system include amino acid sequences set forth in SEQ ID NO:4, SEQ ID NO:5, and SEQ ID NO:6, respectively;
   (2) a heavy chain variable region in which CDR1, CDR2, and CDR3 determined according to the Kabat numbering system include amino acid sequences set forth in SEQ ID NO:7, SEQ ID NO:8, and SEQ ID NO:9, respectively, and a light chain variable region in which CDR1, CDR2, and CDR3 determined according to the Kabat numbering system include amino acid sequences set forth in SEQ ID NO:10, SEQ ID NO:11, and SEQ ID NO: 12, respectively;
   (3) a heavy chain variable region in which CDR1, CDR2, and CDR3 determined according to the Kabat numbering system include amino acid sequences set forth in SEQ ID NO:13, SEQ ID NO:14, and SEQ ID NO:15, respectively, and a light chain variable region in which CDR1, CDR2, and CDR3 determined according to the Kabat numbering system include amino acid sequences set forth in SEQ ID NO:16, SEQ ID NO:17, and SEQ ID NO: 18, respectively;
   (4) a heavy chain variable region in which CDR1, CDR2, and CDR3 determined according to the Kabat numbering system include amino acid sequences set forth in SEQ ID NO:19, SEQ ID NO:20, and SEQ ID NO:21, respectively, and a light chain variable region in which CDR1, CDR2, and CDR3 determined according to the Kabat numbering system include amino acid sequences set forth in SEQ ID NO:22, SEQ ID NO:23, and SEQ ID NO:24, respectively;
   (5) a heavy chain variable region in which CDR1, CDR2, and CDR3 determined according to the Kabat numbering system include amino acid sequences set forth in SEQ ID NO:25, SEQ ID NO:26, and SEQ ID NO:27, respectively, and a light chain variable region in which CDR1, CDR2, and CDR3 determined according to the Kabat numbering system include amino acid sequences set forth in SEQ ID NO:28, SEQ ID NO:29, and SEQ ID NO:30, respectively;
   (6) a heavy chain variable region in which CDR1, CDR2, and CDR3 determined according to the Kabat numbering system include amino acid sequences set forth in SEQ ID NO:31, SEQ ID NO:32, and SEQ ID NO:33, respectively, and a light chain variable region in which CDR1, CDR2, and CDR3 determined according to the Kabat numbering system include amino acid sequences set forth in SEQ ID NO:34, SEQ ID NO:35, and SEQ ID NO:36, respectively;
   (7) a heavy chain variable region in which CDR1, CDR2, and CDR3 determined according to the Kabat numbering system include amino acid sequences set forth in SEQ ID NO:37, SEQ ID NO:38, and SEQ ID NO:39, respectively, and a light chain variable region in which CDR1, CDR2, and CDR3 determined according to the Kabat numbering system include amino acid sequences set forth in SEQ ID NO:40, SEQ ID NO:41, and SEQ ID NO:42, respectively;
   (8) a heavy chain variable region in which CDR1, CDR2, and CDR3 determined according to the Kabat numbering system include amino acid sequences set forth in SEQ ID NO:43, SEQ ID NO:44, and SEQ ID NO:45, respectively, and a light chain variable region in which CDR1, CDR2, and CDR3 determined according to the Kabat numbering system include amino acid sequences set forth in SEQ ID NO:46, SEQ ID NO:47, and SEQ ID NO:48, respectively;
   (9) a heavy chain variable region in which CDR1, CDR2, and CDR3 determined according to the Kabat numbering system include amino acid sequences set forth in SEQ ID NO:49, SEQ ID NO:50, and SEQ ID NO:51, respectively, and a light chain variable region in which CDR1, CDR2, and CDR3 determined according to the Kabat numbering system include amino acid sequences set forth in SEQ ID NO:52, SEQ ID NO:53, and SEQ ID NO:54, respectively;
   (10) a heavy chain variable region in which CDR1, CDR2, and CDR3 determined according to the Kabat numbering system include amino acid sequences set forth in SEQ ID NO:55, SEQ ID NO:56, and SEQ ID NO:57, respectively, and a light chain variable region in which CDR1, CDR2, and CDR3 determined according to the Kabat numbering system include amino acid sequences set forth in SEQ ID NO:58, SEQ ID NO:59, and SEQ ID NO:60, respectively;
   (11) a heavy chain variable region in which CDR1, CDR2, and CDR3 determined according to the Kabat numbering system include amino acid sequences set forth in SEQ ID NO:61, SEQ ID NO:62, and SEQ ID NO:63, respectively, and a light chain variable region in which CDR1, CDR2, and CDR3 determined according to the Kabat numbering system include amino acid sequences set forth in SEQ ID NO:64, SEQ ID NO:65, and SEQ ID NO:66, respectively;
   (12) a heavy chain variable region in which CDR1, CDR2, and CDR3 determined according to the Kabat numbering system include amino acid sequences set forth in SEQ ID NO:67, SEQ ID NO:68, and SEQ ID NO:69, respectively, and a light chain variable region in which CDR1, CDR2, and CDR3 determined according to the Kabat numbering system include amino acid sequences set forth in SEQ ID NO:70, SEQ ID NO:71, and SEQ ID NO:72, respectively;
   (13) a heavy chain variable region in which CDR1, CDR2, and CDR3 determined according to the Kabat numbering system include amino acid sequences set forth in SEQ ID NO:73, SEQ ID NO:74, and SEQ ID NO:75, respectively, and a light chain variable region in which CDR1, CDR2, and CDR3 determined according to the Kabat numbering system include amino acid sequences set forth in SEQ ID NO:76, SEQ ID NO:77, and SEQ ID NO:78, respectively;
   (14) a heavy chain variable region in which CDR1, CDR2, and CDR3 determined according to the Kabat numbering system include amino acid sequences set forth in SEQ ID NO:79, SEQ ID NO:80, and SEQ ID NO:81, respectively, and a light chain variable region in which CDR1, CDR2, and CDR3 determined according to the Kabat numbering system include amino acid sequences set forth in SEQ ID NO:82, SEQ ID NO:83, and SEQ ID NO:84, respectively;
   (15) a heavy chain variable region in which CDR1, CDR2, and CDR3 determined according to the Kabat numbering system include amino acid sequences set forth in SEQ ID NO:85, SEQ ID NO:86, and SEQ ID NO:87, respectively, and a light chain variable region in which CDR1, CDR2, and CDR3 determined according to the Kabat numbering system include amino acid sequences set forth in SEQ ID NO:88, SEQ ID NO:89, and SEQ ID NO:90, respectively;
   (16) a heavy chain variable region in which CDR1, CDR2, and CDR3 determined according to the Kabat numbering system include amino acid sequences set forth in SEQ ID NO:91, SEQ ID NO:92, and SEQ ID NO:93, respectively, and a light chain variable region in which CDR1, CDR2, and CDR3 determined according to the Kabat numbering system include amino acid sequences set forth in SEQ ID NO:94, SEQ ID NO:95, and SEQ ID NO:96, respectively;
   (17) a heavy chain variable region in which CDR1, CDR2, and CDR3 determined according to the Kabat numbering system include amino acid sequences set forth in SEQ ID NO:97, SEQ ID NO:98, and SEQ ID NO:99, respectively, and a light chain variable region in which CDR1, CDR2, and CDR3 determined according to the Kabat numbering system include amino acid sequences set forth in SEQ ID NO:100, SEQ ID NO:101, and SEQ ID NO:102, respectively;
   (18) a heavy chain variable region in which CDR1, CDR2, and CDR3 determined according to the Kabat numbering system include amino acid sequences set forth in SEQ ID NO:103, SEQ ID NO:104, and SEQ ID NO:105, respectively, and a light chain variable region in which CDR1, CDR2, and CDR3 determined according to the Kabat numbering system include amino acid sequences set forth in SEQ ID NO:106, SEQ ID NO: 107, and SEQ ID NO:108, respectively;
   (19) a heavy chain variable region in which CDR1, CDR2, and CDR3 determined according to the Kabat numbering system include amino acid sequences set forth in SEQ ID NO:109, SEQ ID NO:110, and SEQ ID NO:111, respectively, and a light chain variable region in which CDR1, CDR2, and CDR3 determined according to the Kabat numbering system include amino acid sequences set forth in SEQ ID NO:112, SEQ ID NO:113, and SEQ ID NO:114, respectively;
   (20) a heavy chain variable region in which CDR1, CDR2, and CDR3 determined according to the Kabat numbering system include amino acid sequences set forth in SEQ ID NO:115, SEQ ID NO:116, and SEQ ID NO:117, respectively, and a light chain variable region in which CDR1, CDR2, and CDR3 determined according to the Kabat numbering system include amino acid sequences set forth in SEQ ID NO:118, SEQ ID NO:119, and SEQ ID NO:120, respectively;
   (21) a heavy chain variable region in which CDR1, CDR2, and CDR3 determined according to the Kabat numbering system include amino acid sequences set forth in SEQ ID NO:121, SEQ ID NO:122, and SEQ ID NO:123, respectively, and a light chain variable region in which CDR1, CDR2, and CDR3 determined according to the Kabat numbering system include amino acid sequences set forth in SEQ ID NO:124, SEQ ID NO:125, and SEQ ID NO:126, respectively;
   (22) a heavy chain variable region in which CDR1, CDR2, and CDR3 determined according to the Kabat numbering system include amino acid sequences set forth in SEQ ID NO:127, SEQ ID NO:128, and SEQ ID NO:129, respectively, and a light chain variable region in which CDR1, CDR2, and CDR3 determined according to the Kabat numbering system include amino acid sequences set forth in SEQ ID NO:130, SEQ ID NO:131, and SEQ ID NO:132, respectively;
   (23) a heavy chain variable region in which CDR1, CDR2, and CDR3 determined according to the Kabat numbering system include amino acid sequences set forth in SEQ ID NO:133, SEQ ID NO:134, and SEQ ID NO:135, respectively, and a light chain variable region in which CDR1, CDR2, and CDR3 determined according to the Kabat numbering system include amino acid sequences set forth in SEQ ID NO:136, SEQ ID NO:137, and SEQ ID NO:138, respectively;
   (24) a heavy chain variable region in which CDR1, CDR2, and CDR3 determined according to the Kabat numbering system include amino acid sequences set forth in SEQ ID NO:139, SEQ ID NO:140, and SEQ ID NO:141, respectively, and a light chain variable region in which CDR1, CDR2, and CDR3 determined according to the Kabat numbering system include amino acid sequences set forth in SEQ ID NO:142, SEQ ID NO:143, and SEQ ID NO:144, respectively;
   (25) a heavy chain variable region in which CDR1, CDR2, and CDR3 determined according to the Kabat numbering system include amino acid sequences set forth in SEQ ID NO:145, SEQ ID NO:146, and SEQ ID NO:147, respectively, and a light chain variable region in which CDR1, CDR2, and CDR3 determined according to the Kabat numbering system include amino acid sequences set forth in SEQ ID NO:148, SEQ ID NO:149, and SEQ ID NO:150, respectively; and
   (26) a heavy chain variable region in which CDR1, CDR2, and CDR3 determined according to the Kabat numbering system include amino acid sequences set forth in SEQ ID NO:151, SEQ ID NO:152, and SEQ ID NO:153, respectively, and a light chain variable region in which CDR1, CDR2, and CDR3 determined according to the Kabat numbering system include amino acid sequences set forth in SEQ ID NO:154, SEQ ID NO:155, and SEQ ID NO:156, respectively.
[2] The antibody or fragment thereof according to [1], having a heavy chain variable region and a light chain variable region described in any one of the following items (27) to (52):
   (27) a heavy chain variable region including an amino acid sequence set forth in SEQ ID NO:158 and a light chain variable region including an amino acid sequence set forth in SEQ ID NO:160;
   (28) a heavy chain variable region including an amino acid sequence set forth in SEQ ID NO:162 and a light chain variable region including an amino acid sequence set forth in SEQ ID NO:164;
   (29) a heavy chain variable region including an amino acid sequence set forth in SEQ ID NO:166 and a light chain variable region including an amino acid sequence set forth in SEQ ID NO:168;
   (30) a heavy chain variable region including an amino acid sequence set forth in SEQ ID NO:170 and a light chain variable region including an amino acid sequence set forth in SEQ ID NO:172;
   (31) a heavy chain variable region including an amino acid sequence set forth in SEQ ID NO:174 and a light chain variable region including an amino acid sequence set forth in SEQ ID NO:176;
   (32) a heavy chain variable region including an amino acid sequence set forth in SEQ ID NO:178 and a light chain variable region including an amino acid sequence set forth in SEQ ID NO:180;
   (34) a heavy chain variable region including an amino acid sequence set forth in SEQ ID NO:182 and a light chain variable region including an amino acid sequence set forth in SEQ ID NO:184;
   (35) a heavy chain variable region including an amino acid sequence set forth in SEQ ID NO:186 and a light chain variable region including an amino acid sequence set forth in SEQ ID NO:188;
   (36) a heavy chain variable region including an amino acid sequence set forth in SEQ ID NO:190 and a light chain variable region including an amino acid sequence set forth in SEQ ID NO:192;
   (37) a heavy chain variable region including an amino acid sequence set forth in SEQ ID NO:194 and a light chain variable region including an amino acid sequence set forth in SEQ ID NO:196;
   (38) a heavy chain variable region including an amino acid sequence set forth in SEQ ID NO:198 and a light chain variable region including an amino acid sequence set forth in SEQ ID NO:200;
   (39) a heavy chain variable region including an amino acid sequence set forth in SEQ ID NO:202 and a light chain variable region including an amino acid sequence set forth in SEQ ID NO:204;
   (40) a heavy chain variable region including an amino acid sequence set forth in SEQ ID NO:206 and a light chain variable region including an amino acid sequence set forth in SEQ ID NO:208;
   (41) a heavy chain variable region including an amino acid sequence set forth in SEQ ID NO:210 and a light chain variable region including an amino acid sequence set forth in SEQ ID NO:212;
   (42) a heavy chain variable region including an amino acid sequence set forth in SEQ ID NO:214 and a light chain variable region including an amino acid sequence set forth in SEQ ID NO:216;
   (43) a heavy chain variable region including an amino acid sequence set forth in SEQ ID NO:218 and a light chain variable region including an amino acid sequence set forth in SEQ ID NO:220;
   (44) a heavy chain variable region including an amino acid sequence set forth in SEQ ID NO:222 and a light chain variable region including an amino acid sequence set forth in SEQ ID NO:224;
   (45) a heavy chain variable region including an amino acid sequence set forth in SEQ ID NO:226 and a light chain variable region including an amino acid sequence set forth in SEQ ID NO:228;
   (46) a heavy chain variable region including an amino acid sequence set forth in SEQ ID NO:230 and a light chain variable region including an amino acid sequence set forth in SEQ ID NO:232;
   (48) a heavy chain variable region including an amino acid sequence set forth in SEQ ID NO:234 and a light chain variable region including an amino acid sequence set forth in SEQ ID NO:236;
   (49) a heavy chain variable region including an amino acid sequence set forth in SEQ ID NO:238 and a light chain variable region including an amino acid sequence set forth in SEQ ID NO:240;
   (50) a heavy chain variable region including an amino acid sequence set forth in SEQ ID NO:242 and a light chain variable region including an amino acid sequence set forth in SEQ ID NO:244;
   (51) a heavy chain variable region including an amino acid sequence set forth in SEQ ID NO:246 and a light chain variable region including an amino acid sequence set forth in SEQ ID NO:248;
   (52) a heavy chain variable region including an amino acid sequence set forth in SEQ ID NO:250 and a light chain variable region including an amino acid sequence set forth in SEQ ID NO:252;
   (53) a heavy chain variable region including an amino acid sequence set forth in SEQ ID NO:254 and a light chain variable region including an amino acid sequence set forth in SEQ ID NO:256; and
   (54) a heavy chain variable region including an amino acid sequence set forth in SEQ ID NO:258 and a light chain variable region including an amino acid sequence set forth in SEQ ID NO:260.
[3] The antibody or fragment thereof according to Claim 1 or 2,
   in which the antibody or fragment thereof is of IgG1 type and has a mutation in which an asparagine (N) residue as an N-linked glycosylation site in a constant region is deleted or substituted with another amino acid residue.
[4] The antibody or fragment thereof according to any one of [1] to [3],
   in which the antibody or fragment thereof inhibits binding between the spike protein of SARS-CoV-2 and angiotensin-converting enzyme 2 (ACE2).
[5] The antibody or fragment thereof according to any one of [1] to [4],
   in which the antibody or fragment thereof inhibits SARS-CoV-2 infection.
[6] A pharmaceutical composition including:
   the antibody or fragment thereof according to any one of [1] to [5]; and
   a pharmaceutically acceptable carrier.
[7] The pharmaceutical composition according to [6], including:
   two or more kinds of the antibody or fragment thereof according to any one of [1] to [5].

### [Advantageous Effects of Invention]

The neutralizing antibodies created in this study completely inhibited viral infection into cells in an experimental system of infecting susceptible cells with live SARS-CoV-2 virus, when the antibodies were first mixed with the virus and then administered into the cells. In the infection experiment, infection of cells was completely inhibited even at a low concentration of about 3 µg/mL, and it is expected that when these antibodies are actually administered to humans, these antibodies will exhibit high antiviral effects at concentrations in the range ordinary antibody drugs are administered. Therefore, it is expected that such neutralizing antibodies may serve as therapeutic drugs for COVID-19.

### [Brief Description of Drawings]

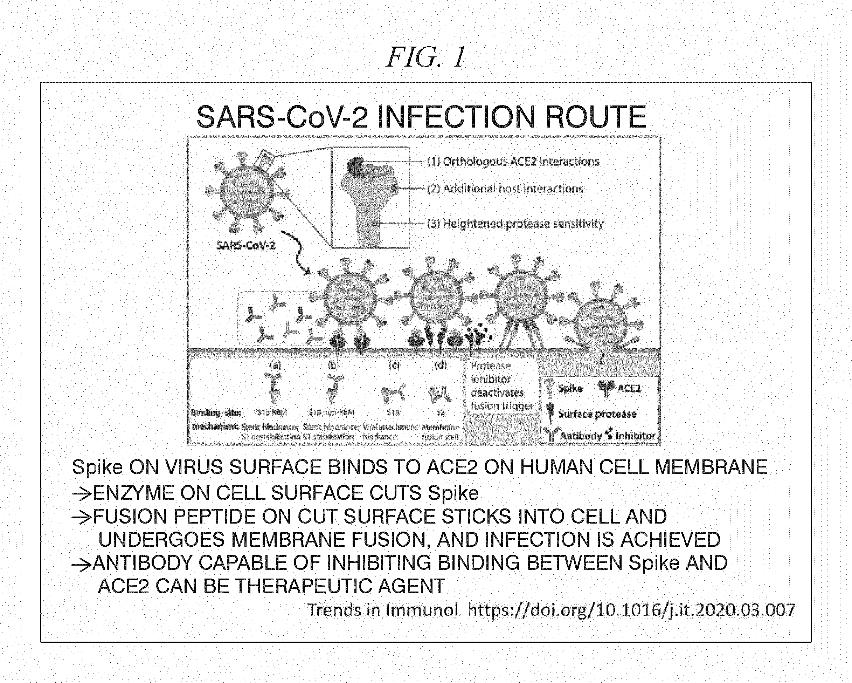

FIG. 1 is a schematic diagram showing an infection route of SARS-CoV-2 virus.
FIG. 2 is a schematic diagram showing a method of screening an antibody.
FIG. 3 is a representative graph representing the results of screening antibodies.
FIG. 4 is a schematic diagram showing a method for obtaining an antibody against the spike protein of SARS-CoV-2.
FIG. 5 is a graph representing the results of quantifying the amount of RNA of viral N protein in oral Swab fluid in Experimental Example 9.
FIG. 6 is a graph representing the results of quantifying the amount of RNA of viral N protein in nasal swab fluid in Experimental Example 9.
FIG. 7 is a diagram representing examples of the three-dimensional structure of an antibody fragment (Fab) bound to SARS-CoV-2 spike protein, as determined in Experimental Example 11.
FIG. 8 is a diagram representing the results of classifying the binding domains of the antibody fragment (Fab), as determined in Experimental Example 11.

### [Description of Embodiments]

### [Antibody or fragment thereof against spike protein of SARS-CoV-2]

According to an embodiment, the present invention provides an antibody against the spike protein of SARS-CoV-2, or a fragment of the antibody. Examples of an antibody fragment include Fab, F(ab')₂, and single-chain Fv (scFv) in which a heavy chain variable region and a light chain variable region are linked by an appropriate linker.

It is preferable that the antibody or fragment thereof according to the present embodiment be a human antibody. A human antibody is preferable because it has a low risk of causing anaphylactic shock even when administered to humans, and a human antimouse antibody (HAMA) does not appear.

The antibody or fragment thereof according to the present embodiment may be such that CDR1, CDR2 and CDR3 of the heavy chain variable region determined according to the Kabat numbering system, and CDR1, CDR2 and CDR3 of the light chain variable region determined according to the Kabat numbering system include the amino acid sequences set forth in the sequence numbers shown in the following Table 1.

**[Table 1]**

| Antibody name | SEQ ID NO. | | | | | |
|---|---|---|---|---|---|---|
| | Heavy chain variable region | | | Light chain variable region | | |
| | CDR1 | CDR2 | CDR3 | CDR1 | CDR2 | CDR3 |
| Ab712 | 1 | 2 | 3 | 4 | 5 | 6 |
| Ab803 | 7 | 8 | 9 | 10 | 11 | 12 |
| Ab816 | 13 | 14 | 15 | 16 | 17 | 18 |
| Ab847 | 19 | 20 | 21 | 22 | 23 | 24 |
| Ab287 | 25 | 26 | 27 | 28 | 29 | 30 |
| Ab326 | 31 | 32 | 33 | 34 | 35 | 36 |
| Ab354 | 37 | 38 | 39 | 40 | 41 | 42 |
| Ab496 | 43 | 44 | 45 | 46 | 47 | 48 |
| Ab376 | 49 | 50 | 51 | 52 | 53 | 54 |
| Ab336 | 55 | 56 | 57 | 58 | 59 | 60 |
| Ab445 | 61 | 62 | 63 | 64 | 65 | 66 |
| Ab159 | 67 | 68 | 69 | 70 | 71 | 72 |
| Ab308 | 73 | 74 | 75 | 76 | 77 | 78 |
| Ab188 | 79 | 80 | 81 | 82 | 83 | 84 |
| Ab175 | 85 | 86 | 87 | 88 | 89 | 90 |
| Ab369 | 91 | 92 | 93 | 94 | 95 | 96 |
| Ab315 | 97 | 98 | 99 | 100 | 101 | 102 |
| Ab302 | 103 | 104 | 105 | 106 | 107 | 108 |
| Ab374 | 109 | 110 | 111 | 112 | 113 | 114 |
| Ab176 | 115 | 116 | 117 | 118 | 119 | 120 |
| Ab114 | 121 | 122 | 123 | 124 | 125 | 126 |
| Ab709TK | 127 | 128 | 129 | 130 | 131 | 132 |
| Ab765 | 133 | 134 | 135 | 136 | 137 | 138 |
| Ab830 | 139 | 140 | 141 | 142 | 143 | 144 |
| Ab863 | 145 | 146 | 147 | 148 | 149 | 150 |
| Ab864 | 151 | 152 | 153 | 154 | 155 | 156 |

The antibody or fragment thereof according to the present embodiment may be such that CDR1, CDR2, and CDR3 of the heavy chain variable region determined according to the IMGT numbering system (http://www.imgt.org/IMGTindex/CDR.php), and CDR1 and CDR3 of the light chain variable region determined according to the IMGT numbering system include the amino acid sequences set forth in the sequence numbers shown in the following Table 2, and CDR2 of the light chain variable region includes the amino acid sequence shown in the following Table 2.

**[Table 2]**

| Antibody name | Heavy chain variable region | | | Light chain variable region | | |
|---|---|---|---|---|---|---|
| | SEQ ID NO. | | | SEQ ID NO. | | Amino acid sequence |
| | CDR1 | CDR2 | CDR3 | CDR1 | CDR3 | CDR2 |
| Ab712 | 261 | 262 | 263 | 264 | 265 | GKT |
| Ab803 | 266 | 267 | 268 | 269 | 270 | AAS |
| Ab816 | 271 | 272 | 273 | 274 | 275 | KAS |
| Ab847 | 276 | 277 | 278 | 279 | 280 | GAS |
| Ab287 | 281 | 282 | 283 | 284 | 285 | GAS |
| Ab326 | 286 | 287 | 288 | 289 | 290 | AAS |
| Ab354 | 291 | 292 | 293 | 294 | 295 | EVS |
| Ab496 | 296 | 297 | 298 | 299 | 300 | DAS |
| Ab376 | 301 | 302 | 303 | 304 | 305 | AAS |
| Ab336 | 306 | 307 | 308 | 309 | 310 | KAS |
| Ab445 | 311 | 312 | 313 | 314 | 315 | DAS |
| Ab159 | 316 | 317 | 318 | 319 | 320 | AAS |
| Ab308 | 321 | 322 | 323 | 324 | 325 | GAS |
| Ab188 | 326 | 327 | 328 | 329 | 330 | WAS |
| Ab175 | 331 | 332 | 333 | 334 | 335 | AAS |
| Ab369 | 336 | 337 | 338 | 339 | 340 | DDS |
| Ab315 | 341 | 342 | 343 | 344 | 345 | DTN |
| Ab302 | 346 | 347 | 348 | 349 | 350 | AAS |
| Ab374 | 351 | 352 | 353 | 354 | 355 | DAS |
| Ab176 | 356 | 357 | 358 | 359 | 360 | GNS |
| Ab114 | 361 | 362 | 363 | 364 | 365 | GAS |
| Ab709TK | 366 | 367 | 368 | 369 | 370 | EDT |
| Ab765 | 371 | 372 | 373 | 374 | 375 | EVS |
| Ab830 | 376 | 377 | 378 | 379 | 380 | AAS |
| Ab863 | 381 | 382 | 383 | 384 | 385 | GAS |
| Ab864 | 386 | 387 | 388 | 389 | 390 | TAS |

The antibody or fragment thereof of the present embodiment may have a heavy chain variable region (VH) including the base sequence or amino acid sequence set forth in the sequence number shown in the following Table 3, and a light chain variable region (VL) including the base sequence or amino acid sequence set forth in sequence number shown in the following Table 3.

**[Table 3]**

| Antibody name | SEQ ID NO. | | | | L chain isotype |
|---|---|---|---|---|---|
| | VH base sequence | VH amino acid sequence | VL base sequence | VL amino acid sequence | |
| Ab712 | 157 | 158 | 159 | 160 | λ |
| Ab803 | 161 | 162 | 163 | 164 | κ |
| Ab816 | 165 | 166 | 167 | 168 | κ |
| Ab847 | 169 | 170 | 171 | 172 | κ |
| Ab287 | 173 | 174 | 175 | 176 | K |
| Ab326 | 177 | 178 | 179 | 180 | K |
| Ab354 | 181 | 182 | 183 | 184 | λ |
| Ab496 | 185 | 186 | 187 | 188 | K |
| Ab376 | 189 | 190 | 191 | 192 | K |
| Ab336 | 193 | 194 | 195 | 196 | K |
| Ab445 | 197 | 198 | 199 | 200 | κ |
| Ab159 | 201 | 202 | 203 | 204 | K |
| Ab308 | 205 | 206 | 207 | 208 | K |
| Ab188 | 209 | 210 | 211 | 212 | K |
| Abl75 | 213 | 214 | 215 | 216 | κ |
| Ab369 | 217 | 218 | 219 | 220 | λ |
| Ab315 | 221 | 222 | 223 | 224 | λ |
| Ab302 | 225 | 226 | 227 | 228 | K |
| Ab374 | 229 | 230 | 231 | 232 | K |
| Ab176 | 233 | 234 | 235 | 236 | λ |
| Ab114 | 237 | 238 | 239 | 240 | K |
| Ab709TK | 241 | 242 | 243 | 244 | λ |
| Ab765 | 245 | 246 | 247 | 248 | λ |
| Ab830 | 249 | 250 | 251 | 252 | K |
| Ab863 | 253 | 254 | 255 | 256 | K |
| Ab864 | 257 | 258 | 259 | 260 | κ |

An antibody or a fragment thereof including, instead of the above-described amino acid sequences, an amino acid sequence obtained by substitution, deletion, insertion, and/or addition of one or a plurality of amino acids of the above-described amino acid sequence, the antibody or fragment thereof being capable of binding to the spike protein of SARS-CoV-2, is also included in the antibody or fragment thereof of the present embodiment. Here, "one or a plurality" may be, for example, 1 to 10, may be 1 to 5, may be 1 to 3, or may be 1 or 2.

Furthermore, an antibody or a fragment thereof including, instead of the above-described amino acid sequences, an amino acid sequence having high sequence identity with the above-described amino acid sequence, the antibody or fragment thereof being capable of binding to the spike protein of SARS-CoV-2, is also included in the antibody or fragment thereof of the present embodiment. Here, "high sequence identity" may be, for example, 80% or more, may be 90% or more, may be 95% or more, may be 97% or more, or may be 99% or more.

As will be described below in the Examples, the antibody or fragment thereof of the present embodiment can bind to the spike protein of SARS-CoV-2 with very high affinity. Therefore, it can also be utilized as a reagent for detecting the spike protein of SARS-CoV-2.

It is preferable that the antibody or fragment thereof of the present embodiment be of IgG1 type and have a mutation of deletion or substitution with another amino acid residue of an asparagine (N) residue, which is an N-linked glycosylation site in a constant region. Examples of another amino acid residue include an alanine (A) residue. The asparagine residue may be conserved and may be referred to as the 297th asparagine residue (N297). Then, the substitution of the asparagine residue with an alanine residue may be referred to as N297A mutation.

The amino acid sequence of the IgG1 constant region having the N297A mutation is set forth in SEQ ID NO:391. The 179th amino acid residue (A) in SEQ ID NO:391 corresponds to the 297th amino acid residue. As will be described later in the Examples, IgG1 having a mutation at N297 suppresses antibody-dependent enhancement (ADE) of infection. Therefore, after the administered antibody binds to SARS-CoV-2, the risk of being taken up into cells and infecting the cells is reduced by ADE.

It is preferable that the antibody or fragment thereof of the present embodiment inhibit binding between the spike protein of SARS-CoV-2 and angiotensin-converting enzyme 2 (ACE2). Such an antibody can be administered to humans to suppress or inhibit infection by SARS-CoV-2. The NCBI Accession Numbers of human ACE2 protein are NP_001358344.1, NP_001373188.1, NP_001373189.1, and the like.

### [Pharmaceutical composition]

According to an embodiment, the present invention provides a pharmaceutical composition including the above-mentioned antibody or fragment thereof and a pharmaceutically acceptable carrier. By administering the pharmaceutical composition of the present embodiment, SARS-CoV-2 infection can be inhibited, and COVID-19 can be prevented or treated.

It is preferable that the pharmaceutical composition of the present embodiment be formulated as an injectable preparation or a preparation for intravenous drip infusion. With regard to the pharmaceutical composition of the present embodiment, examples of the pharmaceutically acceptable carrier include solvents for an injectable preparation or a preparation for intravenous drip infusion.

The solvent may be, for example, an isotonic solution including adjuvants such as physiological saline, glucose, D-sorbitol, D-mannose, D-mannitol, and sodium chloride. The solvent for an injectable preparation may contain alcohols such as ethanol; polyalcohols such as propylene glycol and polyethylene glycol; nonionic surfactants such as Polysorbate 80 (trademark) and HCO-50; and the like.

The pharmaceutical composition may include other additives. Examples of the other additives include a stabilizer, a thickening agent, and a pH adjuster.

Examples of the stabilizer include amino acids such as L-histidine and L-histidine hydrochloride hydrate; parahydroxybenzoic acid esters such as methylparaben and propylparaben; alcohols such as chlorobutanol; and phenols such as phenol and cresol.

Examples of the thickening agent include xanthan gum, sodium alginate, polyvinyl alcohol, hydroxyethylcellulose, sodium polyacrylate, carrageenan, sodium carboxymethylcellulose, and polyvinylpyrrolidone.

Examples of the pH adjuster include phthalic acid, phosphoric acid, citric acid, succinic acid, acetic acid, fumaric acid, malic acid, carbonic acid; potassium salts, sodium salts, or ammonium salts of those acids; and sodium hydroxide.

The pharmaceutical composition can be formulated by appropriately combining the above-described carriers and additives and admixing them in a unit dosage form that is required in generally accepted pharmaceutical practice.

The pharmaceutical composition of the present embodiment may include two or more kinds of the above-described antibodies or fragments thereof. By including two or more kinds of antibodies or fragments thereof that recognize different epitopes on the spike protein of SARS-CoV-2, the possibility is increased that even when a mutation occurs in the spike protein, any of the antibodies or fragments thereof may bind to the spike protein and inhibit SARS-CoV-2 infection.

When the pharmaceutical composition of the present embodiment includes two or more kinds of the above-described antibodies or fragments thereof, it is preferable that these two or more kinds of antibodies or fragments thereof constitute a combination in which the antibodies or fragments thereof do not compete with each other in binding to the SARS-CoV-2 spike protein.

Specific examples of such a combination of the antibodies or fragments thereof include, for example, a combination of Ab159 and Ab765, Ab816, Ab847, Ab863 or Ab864; a combination of Ab188 and Ab765, Ab847, Ab863 or Ab864; a combination of Ab709 and Ab765, Ab847, Ab863 or Ab864; a combination of Ab712 and Ab765, Ab847, Ab863 or Ab864; a combination of Ab765 and Ab803, Ab830 or Ab863; a combination of Ab803 and Ab847, Ab863 or Ab864; a combination of Ab816 and Ab863; a combination of Ab830 and Ab847, Ab863 or Ab864; a combination of Ab847 and Ab863; and a combination of Ab863 and Ab864, as the antibody names shown in the above-described Tables 1 to 3. As will be described below in the Examples, a combination of these antibodies or fragments thereof can simultaneously bind to the SARS-CoV-2 spike protein.

Administration of the pharmaceutical composition of the present embodiment to a patient can be carried out by any appropriate method known to those ordinarily skilled in the art, such as intraarterial injection, intravenous injection, subcutaneous injection, intramuscular injection, and intravenous drip infusion. The dosage varies depending on the body weight and age of the patient, the symptoms of the patient, the administration method, and the like; however, a person ordinarily skilled in the art can appropriately select an appropriate dosage. In general, it is considered appropriate to administer, for adults and children aged 12 years or older and weighing 40 kg or more, about 10 mg to 10 g of one kind of antibody or fragment thereof once to several times by intravenous drip infusion, subcutaneous injection, or intramuscular injection.

### [Other embodiments]

According to an embodiment, the present invention provides a method for preventing or treating COVID-19, the method including administering an effective amount of the above-described antibody or fragment thereof, or the above-described pharmaceutical composition, to a subject. Prevention of COVID-19 can also be referred to as inhibition of SARS-CoV-2 infection.

According to an embodiment, the present invention provides the above-described antibody or fragment thereof for the prevention or treatment of COVID-19.

According to an embodiment, the present invention provides use of the above-described antibody or fragment thereof for the production of a prophylactic agent or therapeutic agent for COVID-19.

### [Examples]

Next, the present invention will be described in more detail by way of Examples; however, the present invention is not intended to be limited to the following Examples.

### [Experimental Example 1]

### (Acquisition of antibodies against spike protein of SARS-CoV-2)

Non-Patent Document 3 discloses a method for using an antigen as "bait" and acquiring memory B cells specific to that antigen. Furthermore, in regard to the acquisition of neutralizing antibodies for SARS-CoV-2, as described in Non-Patent Documents 6 to 12, a trimeric spike protein, a spike protein extracellular domain, and a spike protein RBD have been used as "bait".

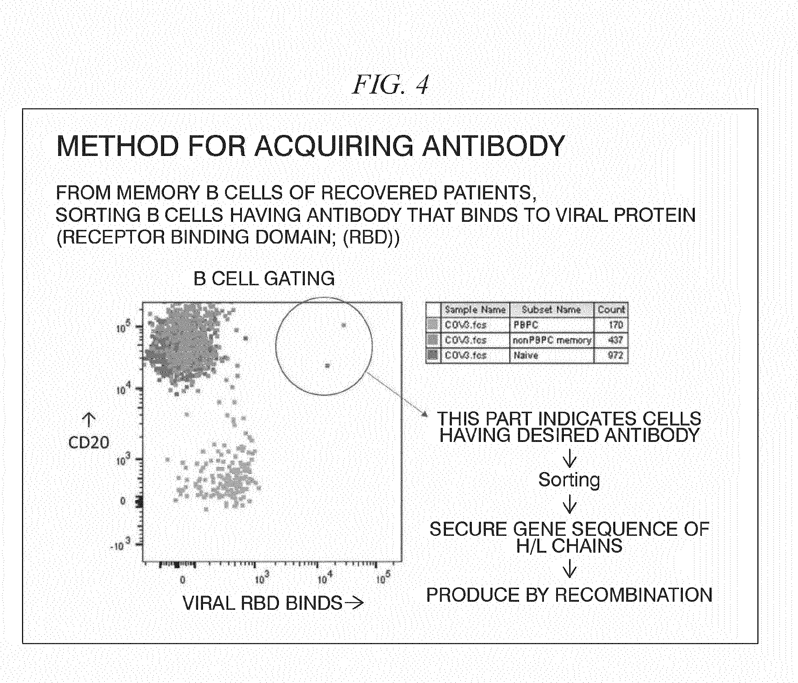

In the present Experimental Example, B cells that produce antibodies against the spike protein of SARS-CoV-2 were sorted by the method shown in FIG. 4, from the peripheral blood lymphocytes of patients who had recovered from COVID-19. Subsequently, antibody genes were acquired from the obtained B cells.

First, a receptor-binding domain (RBD) derived from SARS-CoV-2 (Wuhan strain) and an S1 domain including the RBD were each produced as two types of recombinant proteins, and the recombinant proteins were each fluorescently labeled. Subsequently, these recombinant proteins were allowed to bind to peripheral blood lymphocytes of patients who had recovered from COVID-19, and memory B cells and plasma cells recognized for binding were collected by sorting. Furthermore, antibodies assigned with numbers after 700 in the antibody name in the present specification were acquired by collecting B cells that could bind to both RBD derived from SARS-CoV-2 (Wuhan strain) and RBD derived from SARS-CoV-2 (Brazilian strain) by sorting.

Since RBD is a receptor-binding domain, it is needless to say that the RBD is important for acquiring antibodies against the spike protein of SARS-CoV-2; however, since only a partial region of a large protein is extracted, there is a possibility that the RBD may no longer be a native structure. Furthermore, exposure of an epitope which should have been hidden in the native spike protein may lead to the pickup of even an antibody that binds to the epitope, and there is a possibility of a decrease in efficiency. Thus, S1 protein, which is a larger protein including RBD, was also used as "bait", and an antibody that reacts with a receptor-binding site closer to the native form than simple RBD alone was also selected.

In the above-described technique using "bait", only those memory B cells that have membrane-type antibodies on the cell membrane can be targeted for sorting, and there is a risk of missing plasma cells that do not have membrane-type antibodies and produce secretory antibodies. Therefore, the present inventors also included plasma cells as an object of sorting, in addition to "bait"-bound memory B cells.

Subsequently, the cDNAs of the variable regions of antibody heavy chain and light chain were cloned from each one of the collected cells. Methods for cloning antibody variable regions from single cells include the methods disclosed in Non-Patent Document 3 and Non-Patent Document 4; however, in order to clone variable regions more efficiently, RNA was purified from single-cell sorted cells for the first time by referring to the technique disclosed in Non-Patent Document 5, and then a cDNA library was constructed therefrom. Furthermore, the conditions and primers for PCR amplification of the variable regions from the cDNA library were optimized, and the variable regions could be obtained with higher probability.

Subsequently, the cDNAs of the variable regions of the cloned antibody heavy chain and antibody light chain were introduced into a vector including an expression construct of the antibody heavy chain constant region and a vector including an expression construct of the antibody light chain constant region, respectively, to be expressed, and recombinant antibodies were obtained.

The above-described Table 1 to Table 3 show the antibody names of the acquired representative recombinant antibodies, the sequence numbers of the base sequences and amino acid sequences of the heavy chain variable regions, the sequence numbers of the base sequences and amino acid sequences of the light chain variable regions, the light chain isotypes, the sequence numbers of the amino acid sequences of the heavy chain variable region CDR1, CDR2, and CDR3 as determined according to the Kabat numbering system, the sequence numbers of the amino acid sequences of the light chain variable region CDR1, CDR2, and CDR3 as determined according to the Kabat numbering system, the sequence numbers of the amino acid sequences of the heavy chain variable region CDR1, CDR2, and CDR3 as determined according to the 1MGT numbering system, and the sequence numbers of the amino acid sequences of the light chain variable region CDR1 and CDR3 and the amino acid sequence of CDR2 as determined according to the IMGT numbering system.

### [Experimental Example 2]

### (Screening of antibody 1)

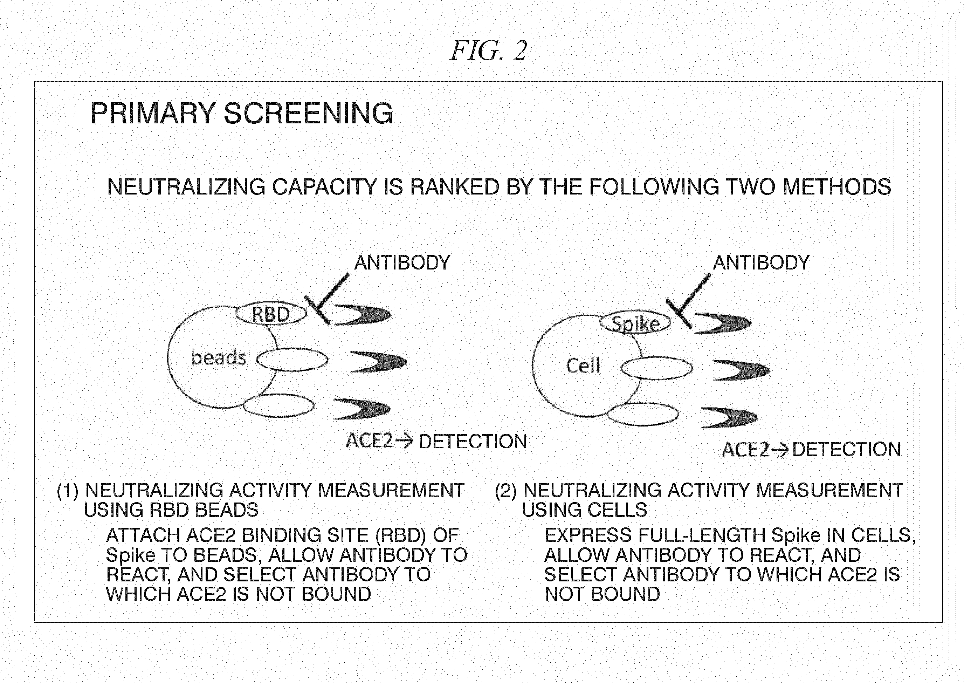

The obtained antibodies were screened by two methods. FIG. 2 is a schematic diagram showing a screening method. In a first screening method, first, RBD-bound beads were reacted with antibodies each prepared at a concentration of 4 µg/mL, 1 µg/mL, or 0.25 µg/mL. Subsequently, fluorescence-labeled soluble ACE2 protein was allowed to react, and flow cytometry was used to examine how much the antibodies compete with ACE2 protein. According to this method, stable results could be obtained due to the use of recombinant proteins. A second screening method will be described later.

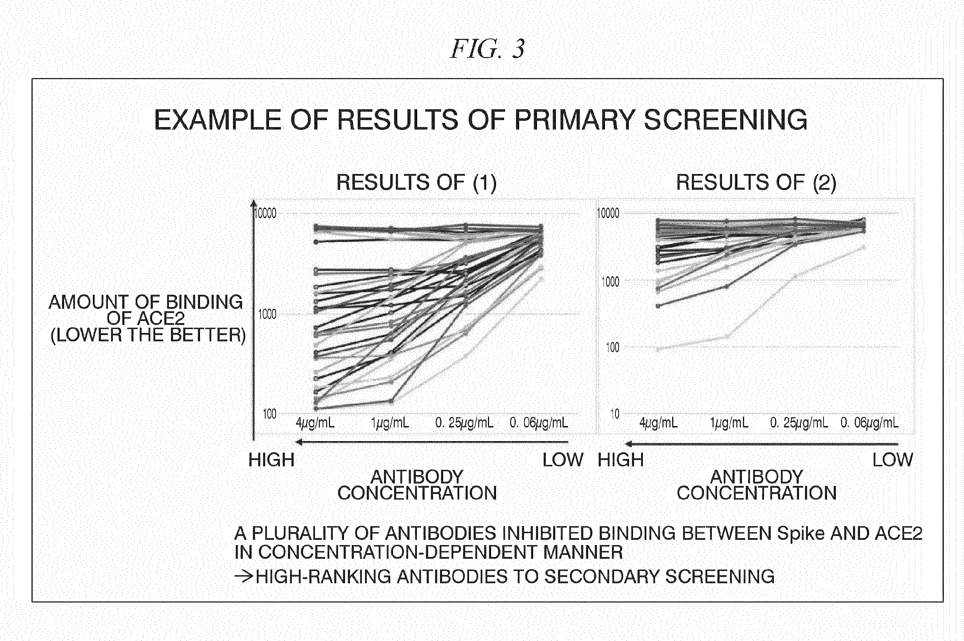

In the present Experimental Example, the first screening method was used. As the RBD, RBD derived from SARS-CoV-2 (Wuhan strain) was used. FIG. 3 is a representative graph showing the results of screening antibodies. Furthermore, the measured values of fluorescence of ACE2 protein are presented in the following Table 4. A smaller value indicates a higher virus-neutralizing activity of the antibody.

**[Table 4]**

| Antibody name | 4µg/mL | 1µg/mL | 0.25µg/mL |
|---|---|---|---|
| Ab287 | 32.6 | 105 | 689 |
| Ab326 | 38.4 | 39.9 | 516 |
| Ab354 | 42.9 | 64.8 | 409 |
| Ab496 | 41.7 | 129 | 2585 |
| Ab376 | 45 | 134 | 445 |
| Ab336 | 106 | 402 | 1113 |
| Ab445 | 142 | 326 | 2197 |
| Ab159 | 78.3 | 114 | 486 |
| Ab308 | 59 | 432 | 1387 |
| Ab188 | 148 | 360 | 1572 |
| Ab175 | 210 | 439 | 1660 |
| Ab369 | 107 | 430 | 1636 |
| Ab315 | 160 | 307 | 927 |
| Ab302 | 189 | 372 | 1405 |
| Ab374 | 244 | 429 | 1362 |
| Ab176 | 366 | 1190 | 2656 |
| Ab114 | 672 | 2036 | 3896 |

### [Experimental Example 3]

### (Screening of antibody 2)

Between the screening methods shown in FIG. 2, a second screening method was used. In the second screening method, first, full-length spike proteins were expressed in 293T cells. As the spike proteins, spike proteins of SARS-CoV-2 (Wuhan strain), α strain, β strain, and γ strain were each expressed.

Subsequently, an antibody whose concentration was adjusted to 4 µg/mL was allowed to react. Subsequently, fluorescence-labeled soluble ACE2 protein was allowed to react to examine how much the antibody competes with ACE2. According to this method, since full-length spike proteins were expressed in cells derived from mammals, a neutralizing capacity closer to that of the native virus could be measured.

FIG. 3 is a representative graph showing the results of screening antibodies. The measured values of fluorescence of ACE2 protein are presented in the following Table 5. Table 5 shows the binding amount (%) of ACE2 protein obtained when ACE2 protein was reacted after the antibody had been reacted, in the case where the binding amount of ACE2 protein obtained when spike protein-expressing cells were reacted with ACE2 protein was taken as 100 (%). A smaller value indicates a higher virus-neutralizing activity of the antibody.

**[Table 5]**

| Antibody name | Wuhan strain | α strain | β strain | γ strain |
|---|---|---|---|---|
| Ab287 | 1.5 | 0.6 | 128.3 | 109.0 |
| Ab326 | 0.2 | 0.3 | 79.1 | 90.9 |
| Ab354 | 0.3 | 0.3 | 133.2 | 113.6 |
| Ab496 | 1.8 | 0.4 | 132.6 | 103.2 |
| Ab376 | 5.1 | - | - | - |
| Ab336 | 5.3 | 4.2 | 116.6 | 106.9 |
| Ab445 | 0.4 | 118.5 | 101.5 | 128.4 |
| Ab159 | 0.5 | 1.9 | 0.5 | 1.5 |
| Ab308 | 3.5 | 7.3 | 105.1 | 87.3 |
| Ab188 | 1.1 | 1.7 | 2.9 | 3.5 |
| Ab175 | 6.9 | - | - | - |
| Ab369 | 4.1 | - | - | - |
| Ab315 | 5.2 | 4.5 | 88.6 | 99.3 |
| Ab302 | 4.7 | 9.2 | 92.6 | 84.6 |
| Ab374 | 10.1 | - | - | - |
| Ab176 | 8.3 | 10.8 | 18.6 | - |
| Ab114 | 10.9 | 4.4 | 7.1 | - |
| Ab709TK | 0.6 | 0.6 | 1.8 | 2.4 |
| Ab712 | 4.6 | 4.4 | 3.8 | 8.1 |
| Ab765 | 0.9 | 2.6 | 1.9 | 3.3 |
| Ab803 | 0.9 | 0.9 | 1.4 | 1.8 |
| Ab816 | 0.3 | 0.5 | 0.4 | 0.7 |
| Ab830 | 0.9 | 1.1 | 4.4 | 6.7 |
| Ab847 | 0.3 | 0.3 | 0.3 | 0.4 |
| Ab863 | 1.7 | 8.3 | 3.4 | 5.3 |
| Ab864 | 0.4 | 0.4 | 1.3 | 2.4 |

### [Experimental Example 4]

### (Live virus neutralization test 1)

For the antibodies screened in Experimental Example 2 or Experimental Example 3, the neutralizing capacity was confirmed in an infection experiment using a live SARS-CoV-2 virus. Measurement of the neutralizing capacity was performed as follows. 100 TCID₅₀/well of SARS-CoV-2 virus (Wuhan strain) was reacted with an antibody, and then the resultant was added to TMPRSS2-expressing Vero E6 cells. Here, "TCID₅₀" represents the median tissue culture infectious dose (50% infective dose). Subsequently, cells were cultured for 5 days to observe cell degeneration, and the minimum concentration at which 100% inhibition of degeneration was observed was measured.

The lowest complete neutralization concentrations thus measured are presented in the following Table 6. As shown in Table 6, a plurality of antibodies were able to completely neutralize live virus at low concentrations.

**[Table 6]**

| Antibody name | Lowest complete neutralization concentration (µg/mL) |
|---|---|
| Ab287 | 0.098 |
| Ab326 | 0.098 |
| Ab354 | 0.098 |
| Ab496 | 0.195 |
| Ab376 | 0.195 |
| Ab336 | 0.391 |
| Ab445 | 0.781 |
| Ab159 | 0.781 |
| Ab308 | 0.781 |
| Ab188 | 0.781 |
| Ab175 | 0.781 |
| Ab369 | 1.563 |
| Ab315 | 1.563 |
| Ab302 | 1.563 |
| Ab374 | 3.125 |
| Ab176 | 3.125 |
| Ab114 | 6.25 |

### [Experimental Example 5]

### (Live virus neutralization test 2)

For the antibodies screened in Experimental Example 2 or Experimental Example 3, the neutralizing capacity was confirmed in an infection experiment using a live SARS-CoV-2 virus. Measurement of the neutralizing capacity was performed as follows. 100 TCID₅₀/well of SARS-CoV-2 virus was reacted with an antibody, and then the resultant was added to TMPRSS2-expressing Vero E6 cells. SARS-CoV-2 (Wuhan strain), α strain, β strain, γ strain, and δ strain were each used as the SARS-CoV-2 virus. With regard to the Wuhan strain, α strain, β strain, and γ strain, the cells were immobilized after 20 to 24 hours, ELISA was performed using an antibody against viral N protein, and the antibody concentration (IC₅₀) at which 50% inhibition of infection was observed was calculated. Furthermore, with regard to the δ strain, the cells were immobilized after 4 to 6 days and stained with Crystal Violet, and then the IC₅₀ was calculated.

The measured IC₅₀ (ng/mL) is presented in the following Table 7. In Table 7, "NA" indicates that virus could not be neutralized.

**[Table 7]**

| Antibody name | Wuhan strain | α strain | β strain | γ strain | δ strain |
|---|---|---|---|---|---|
| Ab287 | - | - | - | - | - |
| Ab326 | 70.99 | 1.354 | NA | NA | 8.1 |
| Ab354 | 108.9 | 1.132 | NA | NA | - |
| Ab496 | 42.48 | 0.2596 | 0.5872 | 2266 | - |
| Ab376 | - | - | - | - | - |
| Ab336 | - | - | - | - | - |
| Ab445 | - | - | - | - | - |
| Ab159 | 308.5 | 7.451 | 10.05 | 1.616 | NA |
| Ab308 | - | - | - | - | - |
| Ab188 | 590.3 | 8.419 | 25.8 | 5.662 | 94.2 |
| Ab175 | - | - | - | - | - |
| Ab369 | - | - | - | - | - |
| Ab315 | - | - | - | - | - |
| Ab302 | - | - | - | - | - |
| Ab374 | - | - | - | - | - |
| Ab176 | - | - | - | - | - |
| Ab114 | - | - | - | - | - |
| Ab709TK | 61.9 | 0.3482 | 4.061 | 0.4853 | 9.4 |
| Ab712 | 489.1 | 2.579 | 13.11 | 1.697 | 30.4 |
| Ab765 | 46.83 | 0.6396 | 6.991 | 1.348 | 10.0 |
| Ab803 | 77.62 | 0.8491 | 11.61 | 0.6308 | 17.7 |
| Ab816 | 101.1 | 1.806 | 12.86 | 1.331 | 7.3 |
| Ab830 | 367.6 | 4.306 | 119.3 | 21.18 | - |
| Ab847 | 147.3 | 2.2 | 18.18 | 1.637 | - |
| Ab863 | NA | NA | NA | NA | - |
| Ab864 | 23.28 | 0.6744 | 3.338 | 0.5746 | - |

### [Experimental Example 6]

### (Pseudovirus test)

For the antibodies screened in Experimental Example 2 or Experimental Example 3, the neutralizing capacity was checked in an infection experiment using a pseudovirus. First, plasmid pCMV3_SARS-cov2d19 series produced from plasmid pNL4-3.luc.R-E- (HIV-1 reporter constructs that are Env- and Vpr-, NIH HIV Reagent Program) and a cDNA clone expression plasmid of the open reading frame of SARS-CoV-2 spike gene (codon-optimized, catalog number "VG40589-UT", Sino Biological, Inc.) were introduced into an Expi293 expression system (Thermo Fisher Scientific, Inc.) to obtain a pseudovirus. This pseudovirus had a SARS-CoV-2 spike protein and a luciferase reporter gene. As the spike protein, each of the spike proteins of SARS-CoV-2 (Wuhan strain), α strain, β strain, γ strain, δ strain, and B.1.1.482 strain was used.

Measurement of the neutralizing capacity was performed as follows. 100 TCID₅₀/well of the pseudovirus was reacted with the antibodies, and then the resultant was added to TMPRSS2-expressing Vero E6 cells having ACE2 gene. Subsequently, the cells were lysed, the luciferase activity was measured, and the antibody concentration (IC₅₀) at which 50% inhibition of infection was observed was calculated.

The measured IC₅₀ (ng/mL) is presented in the following Table 8. In Table 8, "NA" indicates that the virus could not be neutralized.

**[Table 8]**

| Antibody name | Wuhan strain | α strain | β strain | γ strain | δ strain | B.1.1.482 strain |
|---|---|---|---|---|---|---|
| Ab287 | - | - | - | - | - | - |
| Ab326 | 30.99 | 49.28 | NA | NA | 18.36 | NA |
| Ab354 | 55.93 | 93.73 | NA | NA | 71.51 | 276.8 |
| Ab496 | 3.622 | 16.87 | NA | NA | 9.839 | 50.57 |
| Ab376 | - | - | - | - | - | - |
| Ab336 | - | - | - | - | - | - |
| Ab445 | - | - | - | - | - | - |
| Ab159 | 8.52 | 11.95 | 5.369 | 3.691 | 130.3 | 23.33 |
| Ab308 | - | - | - | - | - | - |
| Ab188 | 119.2 | 248.1 | 17.93 | 19.26 | 138.7 | 171.2 |
| Ab175 | - | - | - | - | - | - |
| Ab369 | - | - | - | - | - | - |
| Ab315 | - | - | - | - | - | - |
| Ab302 | - | - | - | - | - | - |
| Ab374 | - | - | - | - | - | - |
| Ab176 | - | - | - | - | - | - |
| Ab114 | - | - | - | - | - | - |
| Ab709TK | 71.7 | 235.7 | 5.8 | 3.3 | 58.6 | 171.8 |
| Ab712 | 104.8 | 103.1 | 7.7 | 2.2 | 79.9 | 16.4 |
| Ab765 | 5.0 | 16.6 | 4.5 | 14.4 | 26.9 | 11.7 |
| Ab803 | 46.8 | 402.4 | 10.4 | 10.6 | 63.9 | 144.1 |
| Ab816 | 23.0 | 17.5 | 9.5 | 1.8 | 23.9 | 39.6 |
| Ab830 | 34.9 | 37.4 | 56.9 | 1.1 | 55.4 | 4.2 |
| Ab847 | 40.3 | 82.1 | 10.3 | 50.2 | 42.2 | 54.8 |
| Ab863 | NA | 30.1 | NA | NA | NA | NA |
| Ab864 | 8.4 | 9.8 | 4.4 | 0.0 | 17.2 | 23.4 |

### [Experimental Example 7]

### (ADE evaluation)

For the antibodies screened in Experimental Example 2 or Experimental Example 3, the presence or absence of antibody-dependent enhancement (ADE) of infection was examined.

Each of the antibodies was reacted with SARS-CoV-2 virus by varying the antibody concentration, and subsequently, the resultant was added to each of Raji cells and THP1 cells, which are known not to express ACE2. When the virus was taken up by these cells, ADE was recognized, indicating that the virus was taken up by the cells dependently on the heavy chain constant region (Fc) of the antibody.

It is known that when there is an N297A mutation in the antibody heavy chain constant region, ADE is suppressed. Thus, an antibody into which the N297A mutation was introduced and an antibody into which the N297A mutation was not introduced were produced and examined.

The results of examination of the presence or absence of ADE are presented in the following Table 9. In Table 9, "N297A introduced" indicates the results obtained using an antibody in which the N297A mutation was introduced, and "N297A not introduced" indicates the results obtained using an antibody in which the N297A mutation was not introduced.

**[Table 9]**

| Antibody name | N297A introduced | N297A not introduced |
|---|---|---|
| Ab287 | - | - |
| Ab326 | Without ADE | With ADE |
| Ab354 | Without ADE | With ADE |
| Ab496 | Without ADE | With ADE |
| Ab376 | - | - |
| Ab336 | - | - |
| Ab445 | - | - |
| Ab159 | - | - |
| Ab308 | - | - |
| Ab188 | - | - |
| Ab175 | - | - |
| Ab369 | - | - |
| Ab315 | - | - |
| Ab302 | - | - |
| Ab374 | - | - |
| Ab176 | - | - |
| Ab114 | - | - |
| Ab709TK | - | - |
| Ab712 | Without ADE | Without ADE |
| Ab765 | - | - |
| Ab803 | Without ADE | Without ADE |
| Ab816 | Without ADE | With ADE |
| Ab830 | - | - |
| Ab847 | Without ADE | Without ADE |
| Ab863 | - | - |
| Ab864 | - | - |

### [Experimental Example 8]

### (Kinetics analysis)

For the antibodies screened in Experimental Example 2 or Experimental Example 3, a kinetics analysis against the SARS-CoV-2 spike protein was carried out. For the analysis, a biomolecular interaction analysis system (product name "Octet Systems", Sartorius AG) was used. Specifically, the concentration K_{off} of the antibody that was not bound to the antigen, the concentration Kₒₙ of the antibody that was bound to the antigen, and the equilibrium dissociation constant K_{D}, which is the ratio of these concentrations, were measured. The RBD of the Wuhan strain was used as the antigen. The measured values of K_{D}, K_{off}, and Kₒₙ of each antibody are presented in the following Table 10.

**[Table 10]**

| Antibody name | K_{D} | Kₒₙ | K_{off} |
|---|---|---|---|
| Ab287 | <1.0×10⁻¹² | 3.01×10⁵ | <1.0×10⁻⁷ |
| Ab326 | 5.14×10⁻¹¹ | 3.43×10⁵ | 1.76×10⁻⁵ |
| Ab354 | 7.26×10⁻¹¹ | 2.60×10⁵ | 1.89×10⁻⁵ |
| Ab496 | <1.0×10⁻¹² | 6.95×10⁵ | <1.0×10⁻⁷ |
| Ab376 | 1.10×10⁻¹⁰ | 1.81×10⁵ | 1.98×10⁻⁵ |
| Ab336 | 2.35×10⁻⁰⁹ | 1.22×10⁵ | 2.86×10⁻⁴ |
| Ab445 | 5.93×10⁻¹¹ | 5.68×10⁵ | 3.37×10⁻⁵ |
| Ab159 | 1.24×10⁻⁰⁹ | 7.22×10⁵ | 8.95×10⁻⁴ |
| Ab308 | 6.62×10⁻⁰⁹ | 9.32×10⁴ | 6.16×10⁻⁴ |
| Ab188 | <1.0×10⁻¹² | 2.60×10⁵ | <1.0×10⁻⁷ |
| Ab175 | <1.0×10⁻¹² | 9.63×10⁵ | <1.0×10⁻⁷ |
| Ab369 | 1.97×10⁻¹⁰ | 7.58×10⁴ | 1.50×10⁻⁵ |
| Ab315 | <1.0×10⁻¹² | 1.72×10⁵ | <1.0×10⁻⁷ |
| Ab302 | <1.0×10⁻¹² | 1.53×10⁵ | <1.0×10⁻⁷ |
| Ab374 | <1.0×10⁻¹² | 1.17×10⁵ | <1.0×10⁻⁷ |
| Ab176 | <1.0×10⁻¹² | 8.31×10⁴ | <1.0×10⁻⁷ |
| Ab114 | <1.0×10⁻¹² | 2.12×10⁵ | <1.0×10⁻⁷ |
| Ab709TK | <1.0×10⁻¹² | 2.85×10⁵ | <1.0×10⁻⁷ |
| Ab712 | <1.0×10⁻¹² | 2.21×10⁵ | <1.0×10⁻⁷ |
| Ab765 | <1.0×10⁻¹² | 1.94×10⁵ | <1.0×10⁻⁷ |
| Ab803 | <1.0×10⁻¹² | 2.28×10⁵ | <1.0×10⁻⁷ |
| Ab816 | <1.0×10⁻¹² | 1.58×10⁵ | <1.0×10⁻⁷ |
| Ab830 | <1.0×10⁻¹² | 3.07×10⁵ | <1.0×10⁻⁷ |
| Ab847 | <1.0×10⁻¹² | 9.04×10⁴ | <1.0×10⁻⁷ |
| Ab863 | <1.0×10⁻¹² | 5.71×10⁴ | <1.0×10⁻⁷ |
| Ab864 | 3.90×10⁻⁰⁹ | 2.04×10⁵ | 7.94×10⁻⁴ |

### [Experimental Example 9]

### (Monkey infection experiment)

Monkeys were infected with SARS-CoV-2 virus, and the effects of the antibody administration were evaluated. Specifically, SARS-CoV-2 virus (JP/TY/WK-521/2020 strain) 2×10⁷ TCIC₅₀ was administered to three cynomolgus monkeys (female) in each group through the eyes, nose, mouth, and trachea. Antibodies were intravenously administered the next day. As the antibodies, a mixture of equal amounts of Ab326, Ab354, and Ab496 was administered at a dose of 20 mg/individual, or human IgG1 (control) was administered at a dose of 20 mg/individual.

Nasal Swab fluid and oral Swab fluid were collected on Day 1 (before antibody administration), Day 3, Day 5, and Day 7. Subsequently, on Day 7, the animals were dissected, and lung tissue was obtained.

A sample of the Swab fluid was subjected to RNA extraction, PCR was performed using TaqMan (registered trademark) Fast Virus 1-step Master Mix (Thermo Fisher Scientific, Inc.), and the amount of RNA of viral N protein was quantified.

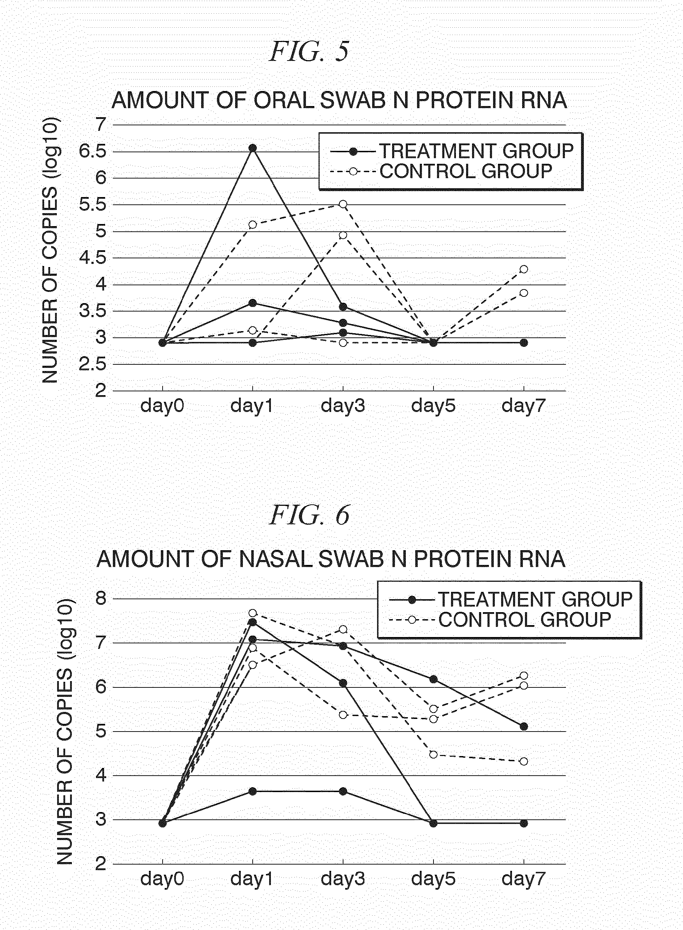

FIG. 5 is a graph showing the results of quantifying the amount of RNA of viral N protein in the oral Swab fluid. Furthermore, FIG. 6 is a graph showing the results of quantifying the amount of RNA of viral N protein in the nasal Swab fluid. In FIG. 5 and FIG. 6, "treatment group" shows the results of a group administered with antibodies obtained by mixing equal amounts of Ab326, Ab354, and Ab496, and "control group" shows the results of a group administered with human IgG1.

As a result, although there were large individual differences, it was clear that the amount of RNA of viral N protein tended to be lower in the treatment group after administration of the antibodies (after Day 3).

Subsequently, another sample of the Swab fluid was serially diluted and reacted with TMPRSS2-expressing Vero E6 cells for 1 hour, and the cells were washed and cultured for 3 days. Degeneration of the cells after culture was assessed, and virus titers were calculated.

The virus titer of the nasal Swab fluid (Log₁₀TCID₅₀/0.1 mL) is presented in the following Table 11. In Table 11, "<" indicates that no infectious virus was recognized. As a result, it was clear that the virus titer of the nasal Swab fluid was reduced in the treatment group as compared with the control group.

**[Table 11**

| Individual number | Group | Day 1 | Day 3 | Day 5 | Day 7 |
|---|---|---|---|---|---|
| 1 | Control group | 1.50 | 1.50 | 0.067 | < |
| 2 | Control group | 2.67 | 0.23 | < | < |
| 3 | Control group | 3.23 | 0.00 | <0.067 | < |
| 4 | Treatment group | 3.00 | < | < | < |
| 5 | Treatment group | 3.33 | < | < | < |
| 6 | Treatment group | < | < | < | < |

A sample of the lung tissue was homogenized, and the virus titer was measured in the same manner as in the case of the Swab fluids. The virus titer (Log₁₀TCID₅₀/1 mL) measured for each lung tissue (Day 7) is presented in the following Table 12. In Table 12, "/" indicates that the individual did not have the tissue. Furthermore, "<" indicates that no infectious virus was recognized. As a result, it was clear that the virus titer of the lung tissue was reduced in the treatment group as compared with the control group.

**[Table 12]**

| Individual number | Group | Right upper lobe | Right middle lobe | Right lower lobe | Left upper lobe | Left middle lobe | Left lower lobe |
|---|---|---|---|---|---|---|---|
| 1 | Control group | < | <1.5 | < | < | < | < |
| 2 | Control group | <0.67 | < | < | < | | < |
| 3 | Control group | < | <1.67 | < | < | < | < |
| 4 | Treatment group | < | < | < | < | < | < |
| 5 | Treatment group | < | < | < | < | < | < |
| 6 | Treatment group | < | < | < | < | | < |

Eight slices of the lung tissue of each individual were produced from another sample of the lung tissue and subjected to hematoxylin and eosin staining, and the extent of lung damage was scored. Scoring was performed according to Ogiwara H, et al., Histopathological evaluation of the diversity of cells susceptible to H5N1 virulent avian influenza virus, Am J Pathol., 184 (1), 171-183, 2014. Specifically, each lung tissue sample was scored according to the following evaluation criteria, and the average value was taken as the lung tissue damage score. The evaluation results of the lung tissue damage score are presented in the following Table 13. As a result, it was clear that the lung tissue damage score was significantly decreased in the treatment group as compared with the control group.

### (Evaluation criteria)

0: Normal lung tissue
1: Mild destruction of the epithelium in the trachea and bronchi
2: Mild inflammatory cell infiltration around bronchioles
3: Moderate inflammatory cell infiltration in alveolar walls and thickening of the alveolar walls
4: Mild alveolar damage accompanied by 10% or less vascular damage
5: Moderate alveolar and vascular damage (11% to 30%)
6: Severe alveolar damage (31% to 50%) accompanied by hyaline membranes and alveolar hemorrhage
7: Severe alveolar damage (51% or more) accompanied by hyaline membranes and alveolar hemorrhage

**[Table 13]**

| Individual number | Group | Lung tissue damage score |
|---|---|---|
| 1 | Control group | 5.38 |
| 2 | Control group | 5.43 |
| 3 | Control group | 5.56 |
| 4 | Treatment group | 5.19 |
| 5 | Treatment group | 4.81 |
| 6 | Treatment group | 4.56 |

### [Experimental Example 10]

### (Hamster infection experiment)

Hamsters were infected with SARS-CoV-2 virus, and the effects of antibody administration were evaluated. Specifically, 1×10³ pfu/100 µL/individual of SARS-CoV-2 virus (UT-NCGM02/Human/2020/Tokyo strain) was administered through the nasal cavity of Syrian hamsters (male, 4 weeks old). Antibodies were intraperitoneally administered the next day. As the antibody, 50 mg/kg body weight of the antibody described in the following Table 14 was administered. In the following Table 14, the control indicates human IgG1 antibody. Subsequently, each hamster was dissected on the fourth day from virus exposure, and lung tissue and blood serum were obtained.

Subsequently, the neutralizing antibody titer in the blood serum was measured for each hamster. Furthermore, RNA was extracted from the lung tissue, PCR was performed, and the amount of viral RNA was quantified.

The neutralizing antibody titer in the blood serum was measured as follows. First, two-fold serial dilutions of blood serum were produced, 35 µL of the blood serum and 35 µL of SARS-CoV-2 virus (140 TCID₅₀) were mixed, and the mixture was incubated at room temperature for 1 hour. Subsequently, 50 µL of the mixed liquid of blood serum and virus was added to confluent TMPRSS2-expressing Vero E6 cells in a 96-well plate, and the cells were incubated at 37°C for 1 hour. Subsequently, 50 µL/well each of DMEM including 5% fetal calf serum (FCS) was added, and the cells were cultured at 37°C for another 3 days. Subsequently, degeneration of the cells was observed with a microscope, and the maximum dilution ratio at which 100% inhibition of degeneration was observed was designated as neutralizing antibody titer.

The neutralizing antibody titer and the Ct value of quantitative RT-PCR for each hamster are presented in the following Table 14. A smaller Ct value indicates that a larger amount of viral RNA is present in the lung tissue.

As a result, it was clear that in the hamsters administered with antibodies, the amount of viral RNA in the lung tissue was markedly reduced as compared to the control group.

**[Table 14]**

| Individual number | Antibody | Neutralizing antibody titer | qRT-PCT (Ct value) |
|---|---|---|---|
| 1 | Control | <10 | 12.86 |
| 2 | Control | <10 | 13.07 |
| 3 | Control | <10 | 13.06 |
| 4 | Ab287 | 1280 | 17.15 |
| 5 | Ab287 | 2560 | 22.46 |
| 6 | Ab287 | 2560 | 17.22 |
| 7 | Ab326 | 1280 | 16.4 |
| 8 | Ab326 | 1280 | 14.31 |
| 9 | Ab326 | 1280 | 14.71 |
| 10 | Ab326 | 1280 | 20.08 |
| 11 | Ab354 | 640 | 17.8 |
| 12 | Ab354 | 1280 | 14.35 |
| 13 | Ab445 | 160 | 16.65 |
| 14 | Ab445 | 320 | 16.41 |
| 15 | Ab445 | 320 | 18.43 |
| 16 | Ab445 | 320 | 23.49 |
| 17 | Ab496 | 2560 | 15.29 |
| 18 | Ab496 | >10240 | 21.03 |

### [Experimental Example 11]

### (Structural analysis of Fab-RBD complex by cryo-electron microscopy)

Antibody fragments (Fab) of the antibodies screened in Experimental Example 2 or Experimental Example 3 were produced and allowed to bind to the SARS-CoV-2 spike protein, and structural analysis was performed by cryo-electron microscopy.

First, a Fab protein expression construct for each antibody was produced and transfected into Expi293F cells to express and purify the Fab protein. Subsequently, the SARS-CoV-2 spike protein extracellular region (6P mutant) expressed and purified in the Expi293F strain was mixed with the antibody fragment (Fab), and then an ice-embedded sample (grid) was produced by rapid freezing.

Subsequently, the produced grid was observed using cryo-electron microscopy (product name "Titan Krios G4: 300 kV", Thermo Fisher Scientific, Inc.). Image processing was performed using RELION-3.1.2, and model construction using Coot was carried out using PDB ID: 6VXX as the initial model. Among the amino acid residues constituting the spike protein, amino acid residues having a central atom approaching within 4 Å to the central atom of an amino acid residue in the variable region of an antibody were defined as epitope.

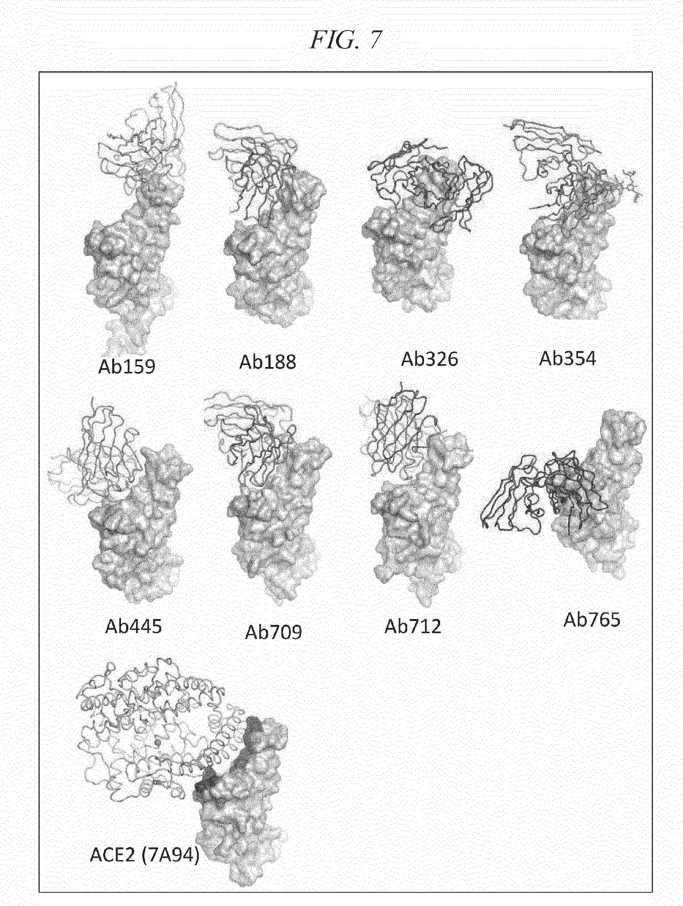

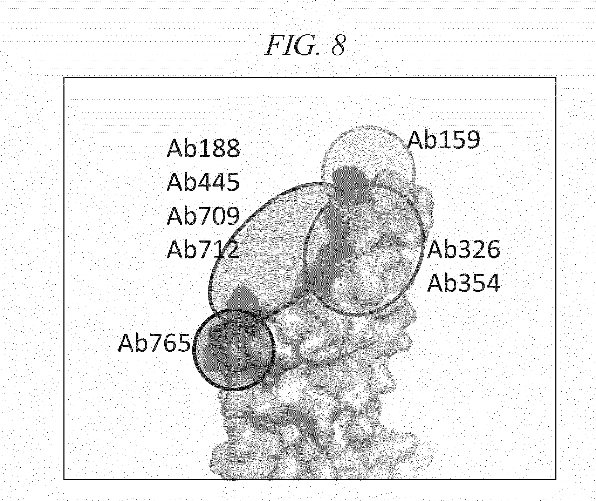

FIG. 7 shows examples of the three-dimensional structure of each antibody fragment (Fab) bound to the SARS-CoV-2 spike protein. As a result, it was clear that the antibody fragment (Fab) binding domain in the RBD overlapped with the ACE2 binding domain. Furthermore, as shown in FIG. 8, it was clear that the binding domain of each antibody fragment (Fab) can be roughly classified into four regions.

The following Tables 15 to 22 show epitopes on the RBD and paratopes on the antibody variable regions as predicted from the results of structural analysis. In Table 15 to Table 22, "RBD" indicates the amino acid residues that constitute the epitope on the RBD, "VH" indicates the amino acid residues that constitute the paratope on the heavy chain variable region, and "VL" indicates the amino acid residues that constitute the paratope on the light chain variable region.

**[Table 15]**

| Ab159 | | | | | | | | | | |
|---|---|---|---|---|---|---|---|---|---|---|
| RBD | S477 | T478 | N481 | E484 | F486 | N487 | Y489 | | | |
| VH | Q1 | V2 | T28 | A32 | D104 | 1105 | L106 | A111 | P113 | Y114 |
| VL | F46 | Y49 | Q55 | | | | | | | |

**[Table 16]**

| Ab188 | | | | | | | | | | | | | | |
|---|---|---|---|---|---|---|---|---|---|---|---|---|---|---|
| RBD | Y449 | F456 | A475 | G476 | S477 | E484 | G485 | F486 | N487 | Y489 | Q493 | S494 | G496 | |
| VH | T28 | 130 | N31 | K33 | Y50 | S52 | D56 | A57 | Y59 | N77 | G101 | Y102 | G105 | E106 |
| VL | Y31 | Y38 | Y98 | S99 | P100 | P102 | | | | | | | | |

**[Table 17]**

| Ab326 | | | | | | | | | | | |
|---|---|---|---|---|---|---|---|---|---|---|---|
| RBD | Y449 | L452 | T470 | Q471 | N481 | G482 | V483 | E484 | F486 | Y489 | F490 |
| VH | H31 | Y32 | V50 | S52 | Y53 | N57 | H59 | T101 | 1103 | R104 | G105 |
| VL | Y32 | S56 | Y92 | R96 | | | | | | | |

**[Table 18]**

| Ab354 | | | | | | | | | | | | | | | |
|---|---|---|---|---|---|---|---|---|---|---|---|---|---|---|---|
| RBD | Y449 | L455 | P479 | N481 | E484 | G485 | F486 | Y489 | F490 | Q493 | S494 | | | | |
| VH | F27 | L28 | F29 | T30 | G31 | Y33 | W50 | N52 | N54 | S55 | A57 | S103 | M 104 | R106 | Fuc128 # |
| VL | Y34 | Y93 | N97 | W99 | | | | | | | | | | | |

| | | | | | | | | | | | | | | | |
|---|---|---|---|---|---|---|---|---|---|---|---|---|---|---|---|
| # represents fucose | | | | | | | | | | | | | | | |

**[Table 19]**

| Ab445 | | | | | | | | | | | | |
|---|---|---|---|---|---|---|---|---|---|---|---|---|
| RBD | R403 | D405 | R408 | K417 | Y449 | Y489 | S494 | Y495 | G496 | Q498 | N501 | Y505 |
| VH | N31 | G106 | V107 | M108 | N109 | P110 | | | | | | |
| VL | Y32 | Y49 | D50 | S52 | N53 | D92 | L94 | | | | | |

**[Table 20]**

| Ab709 | | | | | | | | | | | | |
|---|---|---|---|---|---|---|---|---|---|---|---|---|
| RBD | K417 | Y421 | Y449 | L455 | F456 | Y473 | E484 | G485 | F486 | Y489 | Q498 | Y505 |
| VH | Q100 | S101 | F104 | F107 | Y109 | I110 | | | | | | |
| VL | S26 | S27 | S31 | Y50 | R51 | L54 | S68 | G69 | | | | |

**[Table 21]**

| Ab712 | | | | | | | | | | |
|---|---|---|---|---|---|---|---|---|---|---|
| RBD | G416 | K417 | D420 | Y421 | Y453 | L455 | F456 | F486 | Y489 | Q493 |
| VH | N31 | R102 | Y106 | D107 | G108 | S109 | Y112 | | | |
| VL | Y48 | | | | | | | | | |

**[Table 22]**

| Ab765 | | | | | | | | | |
|---|---|---|---|---|---|---|---|---|---|
| RBD | N440 | K444 | V445 | G446 | G447 | N448 | Q498 | P499 | T500 |
| VH | A33 | S52 | Y53 | Q101 | G102 | V105 | | | |
| VL | Y34 | Y93 | Y97 | | | | | | |

### [Experimental Example 12]

### (Examination of epitope overlapping)

Among the antibodies screened in Experimental Example 2 or Experimental Example 3, combinations of two antibodies that can simultaneously bind to the SARS-CoV-2 spike protein were analyzed. For the analysis, a biomolecular interaction analysis system (product name "Octet Systems", Sartorius AG) was used.

The results of the analysis are presented in the following Table 23. Table 23 shows whether binding was possible when the antibody described in the leftmost column of Table 23 was first reacted and then the antibody placed in the right column of Table 23 was reacted. In Table 23, "-" indicates that binding was impossible, "+" indicates that binding was possible, and "±" indicates intermediate results between the two. That is, combinations of antibodies indicated with "+" are capable of binding to the spike protein simultaneously.

**[Table 23]**

| ↓1st | Ab159 | Abl88 | Ab709 | Ab712 | Ab765 | Ab803 | Ab816 | Ab830 | Ab847 | Ab863 | Ab864 |
|---|---|---|---|---|---|---|---|---|---|---|---|
| Ab159 | | - | - | - | + | - | + | - | + | + | + |
| Ab188 | | | - | - | + | - | - | - | + | + | + |
| Ab709 | | | | - | + | - | - | - | + | + | + |
| Ab712 | | | | | + | - | - | - | + | + | + |
| Ab765 | | | | | | + | - | + | - | + | - |
| Ab803 | | | | | | | ± | - | + | + | + |
| Ab816 | | | | | | | | ± | - | + | - |
| Ab830 | | | | | | | | | + | + | + |
| Ab847 | | | | | | | | | | + | - |
| Ab863 | | | | | | | | | | | + |
| Ab864 | | | | | | | | | | | |

### [Experimental Example 13]

### (Examination of spike protein mutation and antibody binding)

The reactivity between the antibodies screened in Experimental Example 2 or Experimental Example 3 and SARS-CoV-2 spike proteins having various amino acid mutations was examined.

First, full-length SARS-CoV-2 spike proteins having amino acid mutations shown in the following Tables 24 to 27 were each expressed in 293T cells. Subsequently, each of the antibodies whose concentration was adjusted to 4 µg/mL was allowed to react. Subsequently, fluorescence-labeled soluble ACE2 protein was allowed to react to examine how much the antibody competes with ACE2.

The evaluation results are shown in the following Tables 24 to 27. In Tables 24 to 27, antibody names are shown in the leftmost column. Furthermore, amino acid mutations in the SARS-CoV-2 spike protein are shown in the top row. The results of one antibody are shown in a horizontal row, and the results of one kind of spike protein mutant are shown in a vertical column. Furthermore, the numbers in the tables indicate the amount of binding of ACE2 protein at the time of antibody reaction, when the amount of binding of ACE2 protein in the case where an antibody was not reacted was taken as 100. A lower number indicates that the antibody bound to the spike protein and inhibited the binding of ACE2 protein.

**[Table 24]**

| | Wu | P337R | L340A | R346S | R403K | E406 W | K417N | K417E | K417T | D420N | N439K | N440K | K444Q | V445A | G446V |
|---|---|---|---|---|---|---|---|---|---|---|---|---|---|---|---|
| Ab287 | 0.5 | 0.7 | 0.6 | 0.5 | 0.8 | 0.4 | 0.3 | 0.4 | 0.3 | 0.5 | 0.4 | 0.5 | 0.5 | 0.3 | 0.7 |
| Ab326 | 0.2 | 0.3 | 0.3 | 0.3 | 0.3 | 0.2 | 0.2 | 0.2 | 0.3 | 0.2 | 0.3 | 0.2 | 0.2 | 0.3 | 0.4 |
| Ab354 | 0.3 | 0.3 | 0.3 | 0.4 | 0.4 | 0.3 | 0.3 | 0.4 | 0.3 | 0.3 | 0.5 | 0.3 | 0.3 | 0.3 | 0.6 |
| Ab376 | 0.5 | 1.3 | 1.5 | 1.0 | 1.6 | 2.8 | 0.5 | 0.8 | 0.7 | 1.8 | 1.1 | 0.9 | 0.7 | 0.6 | 1.2 |
| Ab496 | 1.8 | 2.1 | 1.6 | 1.8 | 87.6 | 53.1 | 102.7 | 110.0 | 77.4 | 1.2 | 3.2 | 1.3 | 1.6 | 1.3 | 4.5 |
| Ab336 | 1.0 | 3.8 | 2.5 | 2.0 | 2.7 | 111.1 | 50.7 | 109.6 | 20.0 | 4.7 | 4.4 | 3.0 | 0.9 | 1.1 | 1.9 |
| Ab159 | 1.0 | 1.0 | 1.3 | 1.4 | 1.5 | 0.4 | 1.0 | 1.3 | 0.8 | 0.7 | 1.8 | 1.2 | 0.9 | 1.0 | 1.3 |
| Ab175 | 2.1 | 5.3 | 2.8 | 2.6 | 4.3 | 1.6 | 19.4 | 29.9 | 7.3 | 9.9 | 3.6 | 4.1 | 3.2 | 2.0 | 1.7 |
| Ab188 | 2.1 | 1.7 | 1.7 | 1.3 | 1.3 | 1.2 | 0.9 | 1.1 | 1.4 | 2.8 | 1.6 | 1.6 | 1.8 | 1.2 | 2.5 |
| Ab308 | 4.1 | 12.9 | 14.1 | 10.3 | 12.1 | 9.0 | 2.0 | 1.4 | 2.2 | 19.8 | 15.2 | 11.7 | 5.2 | 8.6 | 21.8 |
| Ab445 | 0.4 | 0.6 | 0.4 | 0.5 | 0.5 | 0.4 | 0.5 | 0.8 | 0.5 | 0.4 | 0.6 | 0.5 | 0.4 | 0.5 | 0.6 |

**[Table 25]**

| | Y449H | N450D | L452R | Y453F | L455F | F456V | N460K | A475V | G476S | S477N | T478L | T478K | V483A | E484Q | E484K |
|---|---|---|---|---|---|---|---|---|---|---|---|---|---|---|---|
| Ab287 | 0.2 | 0.4 | 2.7 | 0.8 | 0.6 | 0.9 | 0.4 | 0.6 | 0.5 | 0.4 | 0.4 | 0.3 | 1.5 | 148.7 | 95.6 |
| Ab326 | 0.2 | 0.4 | 0.5 | 0.4 | 0.2 | 1.1 | 0.7 | 0.8 | 0.5 | 0.5 | 2.4 | 0.3 | 3.1 | 13.3 | 73.7 |
| Ab354 | 0.3 | 0.6 | 0.5 | 0.5 | 0.2 | 1.7 | 1.4 | 1.6 | 1.3 | 1.0 | 0.2 | 1.2 | 0.5 | 25.1 | 106.3 |
| Ab376 | 0.4 | 0.6 | 1.0 | 0.9 | 1.7 | 22.7 | 1.1 | 1.2 | 0.8 | 1.2 | 0.7 | 1.6 | 1.0 | 2.6 | 25.0 |
| Ab496 | 93.4 | 1.9 | 1.2 | 2.1 | 26.4 | 1.5 | 0.9 | 1.0 | 1.0 | 0.6 | 1.6 | 0.6 | 0.8 | 1.1 | 98.8 |
| Ab336 | 1.0 | 1.1 | 2.9 | 8.7 | 88.4 | 3.4 | 2.2 | 2.1 | 1.5 | 1.4 | 1.3 | 3.0 | 2.7 | 5.8 | 14.9 |
| Ab159 | 0.3 | 1.7 | 1.4 | 1.9 | 0.6 | 20.7 | 1.7 | 2.1 | 6.4 | 68.3 | 60.6 | 81.6 | 4.2 | 0.6 | 0.5 |
| Ab175 | 1.1 | 2.7 | 2.8 | 4.2 | 3.4 | 54.3 | 5.4 | 4.2 | 4.4 | 8.5 | 2.1 | 3.1 | 4.3 | 3.0 | 1.7 |
| Ab188 | 0.9 | 1.7 | 1.9 | 2.8 | 1.8 | 4.2 | 4.4 | 4.8 | 6.7 | 2.9 | 3.0 | 9.7 | 3.4 | 3.3 | 4.4 |
| Ab308 | 6.5 | 4.5 | 79.1 | 11.0 | 6.2 | 5.1 | 17.8 | 8.2 | 4.6 | 4.4 | 5.3 | 11.4 | 67.7 | 148.3 | 96.6 |
| Ab445 | 0.4 | 0.8 | 0.7 | 0.6 | 0.3 | 7.3 | 2.1 | 4.1 | 2.9 | 1.9 | 0.3 | 2.7 | 2.6 | 3.2 | 8.9 |

**[Table 26]**

| | G485D | F486V | N487T | Y489II | F490L | Q493K | S494P | Y495II | N501Y | Y505II | Del69-70 | D80A | D138Y | Del144 |
|---|---|---|---|---|---|---|---|---|---|---|---|---|---|---|
| Ab287 | 3.5 | 0.5 | 0.4 | 0.3 | 14.7 | 0.4 | 0.4 | 0.6 | 0.6 | 0.3 | 0.4 | 0.4 | 0.4 | 0.4 |
| Ab326 | 1.2 | 0.4 | 0.4 | 0.3 | 81.6 | 16.9 | 1.1 | 1.5 | 2.9 | 0.2 | 0.3 | 0.4 | 0.4 | 0.3 |
| Ab354 | 2.0 | 0.5 | 0.5 | 0.6 | 0.8 | 114.5 | 1.5 | 2.5 | 0.7 | 0.4 | 0.7 | 0.6 | 0.6 | 0.5 |
| Ab376 | 0.8 | 2.0 | 0.5 | 2.8 | 0.6 | 1.4 | 1.4 | 1.1 | 4.1 | 0.7 | 0.8 | 0.6 | 0.7 | 0.5 |
| Ab496 | 96.0 | 65.9 | 0.4 | 8.7 | 5.1 | 1.8 | 3.2 | 2.1 | 0.6 | 0.3 | 0.5 | 0.5 | 0.5 | 0.5 |
| Ab336 | 1.3 | 1.2 | 1.0 | 1.9 | 1.5 | 107.4 | 2.1 | 3.8 | 5.6 | 37.1 | 2.1 | 1.7 | 1.4 | 1.4 |
| Ab159 | 105.7 | 99.3 | 28.8 | 76.7 | 2.7 | 0.8 | 2.2 | 4.0 | 0.4 | 0.4 | 1.0 | 1.8 | 1.9 | 1.7 |
| Ab175 | 3.2 | 3.3 | 69.5 | 6.6 | 2.5 | 1.5 | 4.8 | 3.0 | 5.1 | 3.3 | 3.2 | 2.4 | 2.1 | 2.1 |
| Ab188 | 2.0 | 30.9 | 2.0 | 1.9 | 1.8 | 6.2 | 6.1 | 6.4 | 1.7 | 1.9 | 3.6 | 2.7 | 1.5 | 1.7 |
| Ab308 | 8.0 | 13.4 | 4.5 | 6.1 | 98.8 | 16.5 | 8.5 | 11.5 | 6.9 | 8.4 | 9.3 | 7.3 | 6.1 | 6.6 |
| Ab445 | 1.4 | 1.3 | 0.7 | 1.2 | 2.2 | 1.7 | 2.3 | 70.2 | 102.2 | 80.9 | 2.0 | 2.1 | 2.0 | 2.4 |

**[Table 27]**

| | N165Q | R190S | D215G | N234Q | A570D | D614G | P681H | R682Q | A701V | T716I | S982A | T1027I | D1118 H | V1176 F |
|---|---|---|---|---|---|---|---|---|---|---|---|---|---|---|
| Ab287 | 0.5 | 0.6 | 0.8 | 1.5 | 0.7 | 0.5 | 1.1 | 0.4 | 0.6 | 0.5 | 0.6 | 0.9 | 0.4 | 0.5 |
| Ab326 | 0.3 | 0.5 | 0.7 | 0.8 | 0.6 | 0.3 | 0.3 | 0.3 | 0.5 | 0.4 | 0.4 | 0.7 | 0.4 | 0.4 |
| Ab354 | 0.8 | 0.9 | 1.1 | 1.5 | 0.8 | 0.8 | 1.7 | 0.5 | 0.8 | 0.7 | 0.8 | 0.9 | 0.6 | 0.8 |
| Ab376 | 1.0 | 0.7 | 0.9 | 3.6 | 3.9 | 0.7 | 3.4 | 0.5 | 0.7 | 0.6 | 0.8 | 1.2 | 0.7 | 0.9 |
| Ab496 | 0.6 | 0.6 | 0.6 | 0.7 | 0.6 | 0.3 | 0.4 | 0.3 | 0.6 | 0.4 | 0.4 | 1.1 | 0.4 | 0.6 |
| Ab336 | 2.2 | 1.4 | 2.3 | 8.1 | 0.8 | 1.6 | 2.3 | 1.8 | 1.8 | 2.2 | 2.7 | 2.2 | 1.6 | 1.9 |
| Ab159 | 4.5 | 3.5 | 2.1 | 6.2 | 7.2 | 1.3 | 0.5 | 5.2 | 3.9 | 2.1 | 4.5 | 6.6 | 1.9 | 1.5 |
| Ab175 | 2.8 | 2.7 | 4.6 | 6.9 | 1.8 | 2.8 | 5.2 | 2.9 | 2.2 | 3.7 | 3.2 | 2.9 | 3.0 | 2.7 |
| Ab188 | 2.2 | 3.0 | 5.1 | 9.7 | 3.2 | 4.1 | 3.9 | 2.7 | 2.3 | 4.0 | 4.3 | 5.1 | 3.0 | 2.4 |
| Ab308 | 3.4 | 7.8 | 13.9 | 23.0 | 5.5 | 7.9 | 4.7 | 15.4 | 7.3 | 8.6 | 6.9 | 10.8 | 8.0 | 4.3 |
| Ab445 | 2.9 | 2.8 | 2.2 | 5.5 | 1.9 | 1.4 | 2.1 | 2.1 | 2.7 | 2.4 | 1.3 | 3.3 | 2.1 | 1.5 |

### [Industrial Applicability]

Since the SARS-CoV-2 neutralizing antibody of the present invention can be used as a medicine, the present invention has applicability in industries such as antibody drug production.

## Claims

1. An antibody against spike protein of SARS-CoV-2, or a fragment of the antibody, the antibody or fragment thereof comprising a heavy chain variable region and a light chain variable region described in any one of the following items (1) to (26):
(1) a heavy chain variable region in which complementarity-determining regions (CDR) 1, CDR2, and CDR3 determined according to a Kabat numbering system include amino acid sequences set forth in SEQ ID NO:1, SEQ ID NO:2, and SEQ ID NO:3, respectively, and a light chain variable region in which CDR1, CDR2, and CDR3 determined according to the Kabat numbering system include amino acid sequences set forth in SEQ ID NO:4, SEQ ID NO:5, and SEQ ID NO:6, respectively;
(2) a heavy chain variable region in which CDR1, CDR2, and CDR3 determined according to the Kabat numbering system include amino acid sequences set forth in SEQ ID NO:7, SEQ ID NO:8, and SEQ ID NO:9, respectively, and a light chain variable region in which CDR1, CDR2, and CDR3 determined according to the Kabat numbering system include amino acid sequences set forth in SEQ ID NO:10, SEQ ID NO:11, and SEQ ID NO:12, respectively;
(3) a heavy chain variable region in which CDR1, CDR2, and CDR3 determined according to the Kabat numbering system include amino acid sequences set forth in SEQ ID NO:13, SEQ ID NO:14, and SEQ ID NO:15, respectively, and a light chain variable region in which CDR1, CDR2, and CDR3 determined according to the Kabat numbering system include amino acid sequences set forth in SEQ ID NO:16, SEQ ID NO:17, and SEQ ID NO:18, respectively;
(4) a heavy chain variable region in which CDR1, CDR2, and CDR3 determined according to the Kabat numbering system include amino acid sequences set forth in SEQ ID NO:19, SEQ ID NO:20, and SEQ ID NO:21, respectively, and a light chain variable region in which CDR1, CDR2, and CDR3 determined according to the Kabat numbering system include amino acid sequences set forth in SEQ ID NO:22, SEQ ID NO:23, and SEQ ID NO:24, respectively;
(5) a heavy chain variable region in which CDR1, CDR2, and CDR3 determined according to the Kabat numbering system include amino acid sequences set forth in SEQ ID NO:25, SEQ ID NO:26, and SEQ ID NO:27, respectively, and a light chain variable region in which CDR1, CDR2, and CDR3 determined according to the Kabat numbering system include amino acid sequences set forth in SEQ ID NO:28, SEQ ID NO:29, and SEQ ID NO:30, respectively;
(6) a heavy chain variable region in which CDR1, CDR2, and CDR3 determined according to the Kabat numbering system include amino acid sequences set forth in SEQ ID NO:31, SEQ ID NO:32, and SEQ ID NO:33, respectively, and a light chain variable region in which CDR1, CDR2, and CDR3 determined according to the Kabat numbering system include amino acid sequences set forth in SEQ ID NO:34, SEQ ID NO:35, and SEQ ID NO:36, respectively;
(7) a heavy chain variable region in which CDR1, CDR2, and CDR3 determined according to the Kabat numbering system include amino acid sequences set forth in SEQ ID NO:37, SEQ ID NO:38, and SEQ ID NO:39, respectively, and a light chain variable region in which CDR1, CDR2, and CDR3 determined according to the Kabat numbering system include amino acid sequences set forth in SEQ ID NO:40, SEQ ID NO:41, and SEQ ID NO:42, respectively;
(8) a heavy chain variable region in which CDR1, CDR2, and CDR3 determined according to the Kabat numbering system include amino acid sequences set forth in SEQ ID NO:43, SEQ ID NO:44, and SEQ ID NO:45, respectively, and a light chain variable region in which CDR1, CDR2, and CDR3 determined according to the Kabat numbering system include amino acid sequences set forth in SEQ ID NO:46, SEQ ID NO:47, and SEQ ID NO:48, respectively;
(9) a heavy chain variable region in which CDR1, CDR2, and CDR3 determined according to the Kabat numbering system include amino acid sequences set forth in SEQ ID NO:49, SEQ ID NO:50, and SEQ ID NO:51, respectively, and a light chain variable region in which CDR1, CDR2, and CDR3 determined according to the Kabat numbering system include amino acid sequences set forth in SEQ ID NO:52, SEQ ID NO:53, and SEQ ID NO:54, respectively;
(10) a heavy chain variable region in which CDR1, CDR2, and CDR3 determined according to the Kabat numbering system include amino acid sequences set forth in SEQ ID NO:55, SEQ ID NO:56, and SEQ ID NO:57, respectively, and a light chain variable region in which CDR1, CDR2, and CDR3 determined according to the Kabat numbering system include amino acid sequences set forth in SEQ ID NO:58, SEQ ID NO:59, and SEQ ID NO:60, respectively;
(11) a heavy chain variable region in which CDR1, CDR2, and CDR3 determined according to the Kabat numbering system include amino acid sequences set forth in SEQ ID NO:61, SEQ ID NO:62, and SEQ ID NO:63, respectively, and a light chain variable region in which CDR1, CDR2, and CDR3 determined according to the Kabat numbering system include amino acid sequences set forth in SEQ ID NO:64, SEQ ID NO:65, and SEQ ID NO:66, respectively;
(12) a heavy chain variable region in which CDR1, CDR2, and CDR3 determined according to the Kabat numbering system include amino acid sequences set forth in SEQ ID NO:67, SEQ ID NO:68, and SEQ ID NO:69, respectively, and a light chain variable region in which CDR1, CDR2, and CDR3 determined according to the Kabat numbering system include amino acid sequences set forth in SEQ ID NO:70, SEQ ID NO:71, and SEQ ID NO:72, respectively;
(13) a heavy chain variable region in which CDR1, CDR2, and CDR3 determined according to the Kabat numbering system include amino acid sequences set forth in SEQ ID NO:73, SEQ ID NO:74, and SEQ ID NO:75, respectively, and a light chain variable region in which CDR1, CDR2, and CDR3 determined according to the Kabat numbering system include amino acid sequences set forth in SEQ ID NO:76, SEQ ID NO:77, and SEQ ID NO:78, respectively;
(14) a heavy chain variable region in which CDR1, CDR2, and CDR3 determined according to the Kabat numbering system include amino acid sequences set forth in SEQ ID NO:79, SEQ ID NO:80, and SEQ ID NO:81, respectively, and a light chain variable region in which CDR1, CDR2, and CDR3 determined according to the Kabat numbering system include amino acid sequences set forth in SEQ ID NO:82, SEQ ID NO:83, and SEQ ID NO:84, respectively;
(15) a heavy chain variable region in which CDR1, CDR2, and CDR3 determined according to the Kabat numbering system include amino acid sequences set forth in SEQ ID NO:85, SEQ ID NO:86, and SEQ ID NO:87, respectively, and a light chain variable region in which CDR1, CDR2, and CDR3 determined according to the Kabat numbering system include amino acid sequences set forth in SEQ ID NO:88, SEQ ID NO:89, and SEQ ID NO:90, respectively;
(16) a heavy chain variable region in which CDR1, CDR2, and CDR3 determined according to the Kabat numbering system include amino acid sequences set forth in SEQ ID NO:91, SEQ ID NO:92, and SEQ ID NO:93, respectively, and a light chain variable region in which CDR1, CDR2, and CDR3 determined according to the Kabat numbering system include amino acid sequences set forth in SEQ ID NO:94, SEQ ID NO:95, and SEQ ID NO:96, respectively;
(17) a heavy chain variable region in which CDR1, CDR2, and CDR3 determined according to the Kabat numbering system include amino acid sequences set forth in SEQ ID NO:97, SEQ ID NO:98, and SEQ ID NO:99, respectively, and a light chain variable region in which CDR1, CDR2, and CDR3 determined according to the Kabat numbering system include amino acid sequences set forth in SEQ ID NO:100, SEQ ID NO:101, and SEQ ID NO:102, respectively;
(18) a heavy chain variable region in which CDR1, CDR2, and CDR3 determined according to the Kabat numbering system include amino acid sequences set forth in SEQ ID NO:103, SEQ ID NO:104, and SEQ ID NO:105, respectively, and a light chain variable region in which CDR1, CDR2, and CDR3 determined according to the Kabat numbering system include amino acid sequences set forth in SEQ ID NO:106, SEQ ID NO:107, and SEQ ID NO:108, respectively;
(19) a heavy chain variable region in which CDR1, CDR2, and CDR3 determined according to the Kabat numbering system include amino acid sequences set forth in SEQ ID NO:109, SEQ ID NO:110, and SEQ ID NO:111, respectively, and a light chain variable region in which CDR1, CDR2, and CDR3 determined according to the Kabat numbering system include amino acid sequences set forth in SEQ ID NO:112, SEQ ID NO:113, and SEQ ID NO:114, respectively;
(20) a heavy chain variable region in which CDR1, CDR2, and CDR3 determined according to the Kabat numbering system include amino acid sequences set forth in SEQ ID NO:115, SEQ ID NO:116, and SEQ ID NO:117, respectively, and a light chain variable region in which CDR1, CDR2, and CDR3 determined according to the Kabat numbering system include amino acid sequences set forth in SEQ ID NO:118, SEQ ID NO:119, and SEQ ID NO:120, respectively;
(21) a heavy chain variable region in which CDR1, CDR2, and CDR3 determined according to the Kabat numbering system include amino acid sequences set forth in SEQ ID NO:121, SEQ ID NO:122, and SEQ ID NO:123, respectively, and a light chain variable region in which CDR1, CDR2, and CDR3 determined according to the Kabat numbering system include amino acid sequences set forth in SEQ ID NO:124, SEQ ID NO: 125, and SEQ ID NO:126, respectively;
(22) a heavy chain variable region in which CDR1, CDR2, and CDR3 determined according to the Kabat numbering system include amino acid sequences set forth in SEQ ID NO:127, SEQ ID NO:128, and SEQ ID NO:129, respectively, and a light chain variable region in which CDR1, CDR2, and CDR3 determined according to the Kabat numbering system include amino acid sequences set forth in SEQ ID NO:130, SEQ ID NO:131, and SEQ ID NO:132, respectively;
(23) a heavy chain variable region in which CDR1, CDR2, and CDR3 determined according to the Kabat numbering system include amino acid sequences set forth in SEQ ID NO:133, SEQ ID NO:134, and SEQ ID NO:135, respectively, and a light chain variable region in which CDR1, CDR2, and CDR3 determined according to the Kabat numbering system include amino acid sequences set forth in SEQ ID NO:136, SEQ ID NO:137, and SEQ ID NO:138, respectively;
(24) a heavy chain variable region in which CDR1, CDR2, and CDR3 determined according to the Kabat numbering system include amino acid sequences set forth in SEQ ID NO:139, SEQ ID NO:140, and SEQ ID NO:141, respectively, and a light chain variable region in which CDR1, CDR2, and CDR3 determined according to the Kabat numbering system include amino acid sequences set forth in SEQ ID NO:142, SEQ ID NO:143, and SEQ ID NO:144, respectively;
(25) a heavy chain variable region in which CDR1, CDR2, and CDR3 determined according to the Kabat numbering system include amino acid sequences set forth in SEQ ID NO:145, SEQ ID NO:146, and SEQ ID NO:147, respectively, and a light chain variable region in which CDR1, CDR2, and CDR3 determined according to the Kabat numbering system include amino acid sequences set forth in SEQ ID NO:148, SEQ ID NO:149, and SEQ ID NO:150, respectively; and
(26) a heavy chain variable region in which CDR1, CDR2, and CDR3 determined according to the Kabat numbering system include amino acid sequences set forth in SEQ ID NO:151, SEQ ID NO:152, and SEQ ID NO:153, respectively, and a light chain variable region in which CDR1, CDR2, and CDR3 determined according to the Kabat numbering system include amino acid sequences set forth in SEQ ID NO:154, SEQ ID NO:155, and SEQ ID NO:156, respectively.

2. The antibody or fragment thereof according to claim 1, comprising a heavy chain variable region and a light chain variable region described in any one of the following items (27) to (52):
(27) a heavy chain variable region including an amino acid sequence set forth in SEQ ID NO:158 and a light chain variable region including an amino acid sequence set forth in SEQ ID NO:160;
(28) a heavy chain variable region including an amino acid sequence set forth in SEQ ID NO:162 and a light chain variable region including an amino acid sequence set forth in SEQ ID NO:164;
(29) a heavy chain variable region including an amino acid sequence set forth in SEQ ID NO:166 and a light chain variable region including an amino acid sequence set forth in SEQ ID NO:168;
(30) a heavy chain variable region including an amino acid sequence set forth in SEQ ID NO:170 and a light chain variable region including an amino acid sequence set forth in SEQ ID NO:172;
(31) a heavy chain variable region including an amino acid sequence set forth in SEQ ID NO:174 and a light chain variable region including an amino acid sequence set forth in SEQ ID NO:176;
(32) a heavy chain variable region including an amino acid sequence set forth in SEQ ID NO:178 and a light chain variable region including an amino acid sequence set forth in SEQ ID NO:180;
(34) a heavy chain variable region including an amino acid sequence set forth in SEQ ID NO:182 and a light chain variable region including an amino acid sequence set forth in SEQ ID NO:184;
(35) a heavy chain variable region including an amino acid sequence set forth in SEQ ID NO:186 and a light chain variable region including an amino acid sequence set forth in SEQ ID NO:188;
(36) a heavy chain variable region including an amino acid sequence set forth in SEQ ID NO:190 and a light chain variable region including an amino acid sequence set forth in SEQ ID NO:192;
(37) a heavy chain variable region including an amino acid sequence set forth in SEQ ID NO:194 and a light chain variable region including an amino acid sequence set forth in SEQ ID NO:196;
(38) a heavy chain variable region including an amino acid sequence set forth in SEQ ID NO:198 and a light chain variable region including an amino acid sequence set forth in SEQ ID NO:200;
(39) a heavy chain variable region including an amino acid sequence set forth in SEQ ID NO:202 and a light chain variable region including an amino acid sequence set forth in SEQ ID NO:204;
(40) a heavy chain variable region including an amino acid sequence set forth in SEQ ID NO:206 and a light chain variable region including an amino acid sequence set forth in SEQ ID NO:208;
(41) a heavy chain variable region including an amino acid sequence set forth in SEQ ID NO:210 and a light chain variable region including an amino acid sequence set forth in SEQ ID NO:212;
(42) a heavy chain variable region including an amino acid sequence set forth in SEQ ID NO:214 and a light chain variable region including an amino acid sequence set forth in SEQ ID NO:216;
(43) a heavy chain variable region including an amino acid sequence set forth in SEQ ID NO:218 and a light chain variable region including an amino acid sequence set forth in SEQ ID NO:220;
(44) a heavy chain variable region including an amino acid sequence set forth in SEQ ID NO:222 and a light chain variable region including an amino acid sequence set forth in SEQ ID NO:224;
(45) a heavy chain variable region including an amino acid sequence set forth in SEQ ID NO:226 and a light chain variable region including an amino acid sequence set forth in SEQ ID NO:228;
(46) a heavy chain variable region including an amino acid sequence set forth in SEQ ID NO:230 and a light chain variable region including an amino acid sequence set forth in SEQ ID NO:232;
(48) a heavy chain variable region including an amino acid sequence set forth in SEQ ID NO:234 and a light chain variable region including an amino acid sequence set forth in SEQ ID NO:236;
(49) a heavy chain variable region including an amino acid sequence set forth in SEQ ID NO:238 and a light chain variable region including an amino acid sequence set forth in SEQ ID NO:240;
(50) a heavy chain variable region including an amino acid sequence set forth in SEQ ID NO:242 and a light chain variable region including an amino acid sequence set forth in SEQ ID NO:244;
(51) a heavy chain variable region including an amino acid sequence set forth in SEQ ID NO:246 and a light chain variable region including an amino acid sequence set forth in SEQ ID NO:248;
(52) a heavy chain variable region including an amino acid sequence set forth in SEQ ID NO:250 and a light chain variable region including an amino acid sequence set forth in SEQ ID NO:252;
(53) a heavy chain variable region including an amino acid sequence set forth in SEQ ID NO:254 and a light chain variable region including an amino acid sequence set forth in SEQ ID NO:256; and
(54) a heavy chain variable region including an amino acid sequence set forth in SEQ ID NO:258 and a light chain variable region including an amino acid sequence set forth in SEQ ID NO:260.

3. The antibody or fragment thereof according to claim 1 or 2,
wherein the antibody or fragment thereof is of lgG1 type and has a mutation in which an asparagine (N) residue as an N-linked glycosylation site in a constant region is deleted or substituted with another amino acid residue.

4. The antibody or fragment thereof according to any one of claims 1 to 3,
wherein the antibody or fragment thereof inhibits binding between the spike protein of SARS-CoV-2 and angiotensin-converting enzyme 2 (ACE2).

5. The antibody or fragment thereof according to any one of claims 1 to 4, wherein the antibody or fragment thereof inhibits SARS-CoV-2 infection.

6. A pharmaceutical composition comprising:
the antibody or fragment thereof according to any one of claims 1 to 5; and
a pharmaceutically acceptable carrier.

7. The pharmaceutical composition according to claim 6, comprising:
two or more kinds of the antibody or fragment thereof according to any one of claims 1 to 5.
